# EUROPEAN PATENT SPECIFICATION

(11) **EP 4 598 472 B1**
(45) Date of publication and mention of the grant of the patent: **25.03.2026**
(21) Application number: 23800532.6
(22) Date of filing: 05.10.2023
(51) Int. Cl.: A61B 34/30, A61B 34/37, A61B 34/00

(54) **MASTER CONTROL DEVICE FOR SURGICAL TELEOPERATION, SYSTEM FOR TELEOPERATION AND CONTROL METHOD FOR SIMULATIONS**
HAUPTSTEUERGERÄT FÜR DIE CHIRURGISCHE TELEOPERATION, SYSTEM ZUR TELEOPERATION UND STEUERVERFAHREN FÜR SIMULATIONEN
DISPOSITIF MAÎTRE DE COMMANDE POUR TÉLÉOPÉRATION CHIRURGICALE, SYSTÈME DE TÉLÉOPÉRATION ET MÉTHODES DE COMMANDE POUR SIMULATIONS

(30) Priority: 07.10.2022 IT 202200020655
(43) Date of publication of application: 13.08.2025
(73) Proprietor: Medical Microinstruments, Inc., Wilmington, DE 19801 (US)
(72) Inventor: CAPONIGRI, Irene, 56121 Pisa (IT); BAGHERI GHAVIFEKR, Matteo, 56121 Pisa (IT); SIMI, Massimiliano, 56121 Pisa (IT)
(74) Representative: Signori, Ludovico
(86) International application number: PCT/IB2023/059998
(87) International publication number: WO 2024/075054

(56) References cited:
- WO-A1-2019/220407
- WO-A1-2022/175802
- US-B1- 6 594 552

## Description

### . Field of the invention

**.** The present invention relates to a master control device.

**.** The master control device according to the invention is particularly suitable for a system for robotic surgery.

**.** The present invention further relates to a system for medical or surgical teleoperation comprising at least one master control device.

**.** Moreover, the invention relates to a control method for simulating controlling the degree of freedom of opening/closing with modulation of the clamping/cutting force of a slave surgical instrument.

### . Background art

**.** Robotic surgery apparatuses are generally known in the art and typically comprise a central robotic tower or a cart and one or more robotic arms extending from the tower/cart. Each arm comprises a motorized positioning system (or manipulator) for moving a surgical instrument distally attachable thereto, in order to perform surgical procedures on a patient. The patient typically lies on an operating bed located in the operating room, in which sterility is ensured to avoid bacterial contamination due to non-sterile parts of the robotic apparatus.

**.** To control the robotic manipulator and thus the slave surgical instrument, the surgeon acts on one or more master control devices, according to a master-slave teleoperation architecture.

**.** In fact, by means of the opening/closing command of the master control device, it is desirable to control at least two functions of the slave surgical instrument: the degree of freedom of opening/closing between the tips (jaws) of the surgical instrument of the slave device can also perform, with closed tips (jaws), a gripping or cutting function and an increase in the gripping or cutting force delivered by the tips of the slave surgical instrument.

**.** Slave surgical instruments with different degrees of freedom of movement are typically provided in the known robotic systems for surgical teleoperation. Of particular interest is the control of the degree of freedom of opening and closing of the slave terminal clamp, and in particular the gripping capacity with adjustable force by means of a dedicated master controller.

**.** There are several known examples of user-controlled master devices (typically a surgeon) in which the degrees of freedom of movement can be associated with kinematic chains formed by rigid links connected with movable joints which derive position and orientation thereof together with a degree of freedom for managing the opening/closing and modulating the gripping force of tissues, needles or sutures with an extra stroke of the same movement.

**.** In a known configuration, disclosed for example by the prior art document US-6594552, there is provided a master device with differentiated rigidity during the closing operation, in which there is shown an elastic mechanism defining a variation in rigidity associated with the beginning of the step of modulating the gripping force of the slave surgical instrument.

. Otherwise, "flying" master devices have also been suggested, i.e., kinematically not constrained to any element of the master console (unconstrained, groundless), in which the degrees of freedom of opening/closing and gripping can be derived from dedicated internal sensors or, in other solutions, can be derived from poses, positions or orientations of optical sensors tracked in the workspace, for example, by means of one or more cameras.

**.** Some further examples of both master and constrained devices are shown in WO-2019-099584.

**.** According to some known examples, unconstrained, wireless or wired flying masters have been disclosed, in which the degrees of freedom of movement including those of opening/closing and gripping can be derived from detection of poses, positions or orientations of magnetometer-type sensors tracked in the workspace.

**.** In particular, it is known to use a pair of electromagnetic sensors each with 6 degrees of freedom which are mounted on the same right and/or left master device body and having at least one degree of freedom of relative movement, in which that single relative motion along a defined trajectory controls both the degree of opening and closing and the modulation of the gripping force, as shown for example by WO-2019-220408 on behalf of the same Applicant.

**.** For example, WO-2022-175800 of the same Applicant discloses a master control device solution of the type mechanically/kinematically unconstrained to the operating console (groundless or unconstrained according to an English terminology commonly adopted in the field), i.e., a master device of the "flying" type, which is equipped with a pair of sensors or tracking markers in a predeterminable three-dimensional tracking volume which can be generated by an electromagnetic emitter. In particular, such a known solution shows a strategy according to which the system control unit verifies the existence of the predefined geometric relationships between the two sensors, i.e., between two mutually hinged rigid rods of the body of the master control device mounting in sensors.

**.** The well-known master device solutions, while partially advantageous in some respects, are by no means without drawbacks.

**.** As shown for example in the prior art document WO-2019-220407 to the same Applicant, an elastic element can be provided on an unconstrained master device, in the specific case a pre-loaded trigger in cantilevered position between the rods, which is capable of providing force feedback to the surgeon when the opening/closing angle between the rods of the master device is less than a certain predeterminable threshold.

Further prior art is disclosed in WO 2022/175802 A1.

**.** Therefore, in such known solutions, a first angular travel between the rods of the master device commands the enslaved opening/closing of the slave device, while a second angular travel between the rods commands an enslaved increase in gripping force (or cutting force). When opening, this type of solution does not fully solve the problem, because when the surgeon opens the rods, i.e., loosens the grip on the master device so that under the bias of the spring provided between the rods they tend to open, a first angular stroke is responsible for commanding the enslaved loosening of the degree of freedom of gripping (or cutting) of the slave device. Therefore, the surgeon sees the master device open while the slave device remains closed, and for these reasons the surgeon perceives a sort of delay, which is undesirable, between the opening command provided and the behavior of the slave surgical instrument enslaved thereto.

**.** The need is felt to manage together with the position, orientation and opening and closing of a teleoperated robotic surgical gripper, also a modulation of the gripping force.

**.** It is felt by the user who manipulates the master and needs to clearly understand which movement or part of the movement is associated with the closing/opening of the grippers and which is associated with the modulation of the gripping force.

**.** In addition, the virtual control point which identifies the slave device, where it follows a point in the master workspace which is usually calculated as the midpoint between the sensors of the master device mounted on the rods, necessarily imposes some inconsistencies if the master device is not closed symmetrically. In other words, when for example only one of the rods of the master device is moved with respect to the master reference system, then also the midpoint between the rods translates, and this results in an enslaved translation of the control point in the slave space. As a result, it can thus occur that in the face of a closing command given by the surgeon, a closing is observed together with a rotation of the tips (jaws) of the slave surgical instrument, to bring the control point of the slave device to the position commanded by the master command.

**.** Therefore, in such a situation, there is a risk of transmitting an unwanted command to the surgical instrument, in the specific case the joint rotation of the tips, i.e., the involuntary actuation of the yaw joint of the articulated end of the surgical instrument as an effect of the translation of the midpoint between the rods of the master device, i.e., between the sensors with respect to the origin of the master reference system.

**.** Therefore, the need is strongly felt to improve and make more intuitive and precise the control of the degree of freedom of opening/closing of a slave surgical instrument controlled by a master device of the unconstrained type, to allow discriminating an action of opening/closing from an action of increase/decrease of the closing, gripping and/or cutting force between the tips (jaws) of the surgical instrument of the slave device.

**.** The user manipulating the master has felt the need to associate a closing/opening movement of the fingers with a closing/opening movement of the grippers and to associate a movement of greater pressure of the fingers with a modulation of the gripping force.

**.** Meanwhile, the need to avoid, or at least minimize, the risk of transmitting unwanted commands from the master device to the slave device, during the operation of the degree of freedom of opening/closing of the slave surgical instrument, is strongly felt.

**.** The need to measure the opening/closing movement separately from the closing/gripping/cutting force modulation movement in a master device for medical or surgical teleoperation is strongly felt.

**.** The need is felt to adequately measure the opening/closing movement and the force modulation movement with adequate resolution, adequate robustness, a simple and economical design, minimizing the number of sensors or electronic components on board.

### . Solution

. It is an object of the present invention to overcome the drawbacks complained of with reference to the prior art and to provide a solution to the need to provide improved control over the opening and closing of an articulated gripping and/or cutting end of a surgical instrument for medical or surgical teleoperation.

. This and other objects are achieved by a system according to claim **1**, as well as by a control method for simulating a medical or surgical teleoperation according to claim 17, and a program according to claim 18. Surgical methods in the sense of Art. 53(c) EPC do not form part of the claimed invention.

. Some advantageous embodiments are the subject of the dependent claims.

**.** According to an aspect of the invention, a method for controlling a medical or surgical teleoperation comprises the steps of (i) providing at least one master control device for controlling at least one degree of freedom of opening/closing of a slave surgical instrument, said master control device comprising a first tracking sensor and a second tracking sensor; (ii) detecting a first variation of the geometric relationship between the first tracking sensor and the second tracking sensor of the master control device along a first direction, and based on the first variation of the detected geometric relationship, moving the slave surgical instrument to open/close; (iii) detecting a second variation of the geometric relationship between the first tracking sensor and the second tracking sensor of the master control device along a second direction, based on the second variation of the detected geometric relationship, varying the clamping/cutting force of the slave surgical instrument.

**.** The control method is also applicable to a computer simulation of a slave surgical instrument.

**.** According to an aspect of the invention, a computer program is provided which is configured to carry out at least some (and preferably all) of the steps of the control method.

**.** According to an aspect of the invention, a robotic system for medical or surgical teleoperation comprises at least one slave surgical instrument with at least one degree of freedom of opening/closing, and at least one master control device for controlling at least the degree of freedom of opening/closing of the slave surgical instrument, said master control device comprising a first tracking sensor and a second tracking sensor.

**.** According to an aspect of the invention, the system comprises at least one control unit configured to detect a first variation of the geometric relationship between the first tracking sensor and the second tracking sensor of the master control device along a first direction, and based on the first variation of the detected geometric relationship, move a slave surgical instrument to open/close; detect a second variation of the geometric relationship between the first tracking sensor and the second tracking sensor of the master control device along a different second direction, and based on the second variation of the detected geometric relationship, vary the clamping/cutting force of the slave surgical instrument.

**.** According to an implementation, the variation of the geometric relationship between the first tracking sensor and the second tracking sensor is amplified, i.e., multiplied with respect to the variation of the geometric relationship between manipulation interface portions of the master control device, adapted to receive the fingers of an operator.

**.** According to an aspect of the invention, a master control device is adapted to control a degree of freedom of opening/closing of a slave surgical instrument and comprises a body having a first operating portion and a second operating portion which are mutually movable under the action imparted by the hand of a surgeon to take at least one open configuration, adapted to control at least one enslaved open configuration of the slave surgical instrument, and at least one closed configuration, adapted to control at least one enslaved closed configuration of the slave surgical instrument.

**.** According to an aspect of the invention, the at least one closed configuration of the master device comprises a first closed configuration, and a second closed configuration, in which a transition between the first closed configuration and the second closed configuration is adapted to control a variation of the clamping/cutting force of the slave surgical instrument.

**.** The master control device can be of the kinematically unconstrained type to the operating console and tracked by a special tracking system.

**.** According to an aspect of the invention, the master control device further comprises a first tracking sensor in operating connection with the first operating portion of the master device body, and a second tracking sensor (32) in operating connection with the second operating portion (11) of the master device body, in which: (i) a transition between the at least one open configuration and the first closed configuration results in a first variation of the geometric relationship between the first tracking sensor and the second tracking sensor; (ii) a transition between the first closed configuration and the second closed configuration results in a second variation of the geometric relationship between the first tracking sensor and the second tracking sensor; (iii) said first variation of the geometric relationship and said second variation of the geometric relationship occur along two different directions.

**.** The master control device can further comprise a first manipulation portion and a second manipulation portion for the action imparted by the surgeon's hand; in which the first manipulation portion is in operating connection with the first operating portion of the body of the master device, and the second manipulation portion is in operating connection with the second operating portion of the master device body, in which the first manipulation portion and the second manipulation portion are mutually movable along a single third direction, e.g., relative approach/distancing.

**.** According to an aspect of the invention, a robotic system for medical or surgical teleoperation and/or for simulating a teleoperation comprises at least one slave surgical instrument with at least one degree of freedom of opening/closing, and at least one master control device for controlling at least the degree of freedom of opening/closing of the slave surgical instrument, said master control device comprising a first tracking sensor and a second tracking sensor.

**.** The system can comprise at least one control unit, configured to detect pose or movement of the tracking sensors and transfer the movement to at least one slave surgical instrument. The "pose" of the sensor is preferably intended to indicate the position (e.g.: x-y-z coordinates) and orientation (e.g.: roll-pitch-yaw) with respect to a global reference set of three (which can be positioned in the console).

**.** According to an aspect of the invention, the actuation of a control movement imposed by the user to the master control device according to a single trajectory of closing/opening movement determines: the relative movement between the two sensors of the master control device according to a first relative movement direction detected and transferred by the control unit to open and close the slave surgical instrument.

**.** According to an aspect of the invention, the relative movement between the same two sensors of the master control device according to a second relative movement direction detected and transferred by the control unit in only gripping force modulation of the slave surgical instrument.

**.** The relative movement between the two sensors according to a first relative movement direction associated with opening and closing can be a variation of relative distance, and the relative movement between the same two sensors according to a second relative movement direction associated with force modulation alone can be a variation of orientation.

**.** The relative movement between the two sensors according to a first relative movement direction associated with the opening and closing can be a first defined curved trajectory and/or circumferential arc, and the relative movement between the same two sensors according to a second relative movement direction associated with the force modulation only can be a second and different defined curved trajectory and/or circumferential arc.

**.** Said second and different defined curved trajectory of said first relative movement direction can have opposite concavity to said first defined curved trajectory of said second relative movement direction.

**.** The control unit can be configured to inhibit any translation, induced to the slave surgical instrument or the control point, during the step of varying the clamping/cutting force, translation which would be due to a variation in the geometric relationship between the sensors along the first direction X1 (which commands opening/closing of the slave device). In other words, during the modulation of the clamping/cutting force F and/or during the second relative movement between the two sensors 31, 32 according to said second relative movement direction X2, the control unit 104 is configured to inhibit any translation induced to the slave surgical instrument 170 or of a control point 177 caused by any further relative movement between the two sensors 31, 32 according to said first relative movement direction X1.

**.** According to an implementation, by exerting in closing/opening the control movement of said single movement trajectory of said master control device, no translational movements of the slave surgical instrument or of the control point are mechanically transferred and/or the midpoint calculated with the distance or with the relative angle between the sensors is mechanically kept unchanged and constant.

**.** According to an implementation, by exerting the control movement of said single movement trajectory in closing, upon reaching and exceeding said point of contact, further relative movement according to said first relative movement direction is mechanically inhibited, while only movement according to said second relative movement direction is mechanically made possible.

**.** According to an implementation, by exerting in opening the control movement of said single movement trajectory upon reaching and exceeding said point of contact, the relative movement according to said second relative movement direction is mechanically inhibited, while only movement according to said first relative movement direction is mechanically made possible.

**.** According to an implementation, by exerting in closing the control movement of said single movement trajectory, upon reaching and exceeding said point of contact and during movement along said second relative movement direction it is necessary to exert a closing force greater than the force necessary to move along said first relative movement direction.

**.** Said single movement trajectory can be a curve and/or an circumferential arc or is a straight line.

**.** According to an implementation, the first part of the control movement of said single movement trajectory imposed by the user on the master control device is associated with the actuation of said first relative movement direction and therefore of closing/opening of said slave surgical instrument, the second part of the control movement of said single movement trajectory imposed by the user on the master control device is associated with the actuation of said second relative movement direction and therefore of varying the clamping/cutting force of the slave surgical instrument.

**.** According to an implementation, the second variation of the geometric relationship is determined by the elastic deformation of the master device body.

**.** By virtue of the suggested solutions, it is possible to discriminate a first command aimed at opening/closing the slave surgical instrument from a second command aimed at varying the closing force of the same slave surgical instrument, even if said first and second command are determined by the same or similar action by the surgeon on the master device, such as the relative approaching or distancing of two fingers of the surgeon's hand.

**.** By virtue of the suggested solutions, it is possible to manage the opening/closing of a slave robotic surgical grippers and also the modulation of the gripping force (squeeze). Position and orientation management of the slave surgical grippers can also be included by means of the same master control device.

**.** It is therefore possible for the user manipulating the master device to clearly understand which movement or part of movement is associated with the closing/opening of the slave grippers and which is instead associated with the modulation of the gripping force of the slave grippers themselves.

**.** Therefore, the user becomes capable of associating a movement or action of closing/opening the fingers with a movement of closing/opening the grippers, and associating a movement or action of pressing the fingers with a modulation of the gripping force.

**.** It allows adequately measuring the opening/closing movement and the gripping force modulation movement with adequate resolution, robustness, and with a simple and economical design, minimizing the number of sensors on board the master device.

**.** By virtue of the suggested solutions, in an unconstrained master controller, the degrees of freedom of movement thereof, including those of opening/closing and modulation of the gripping force (grip/squeeze) can be derived from poses of only two sensors tracked in the workspace.

**.** By virtue of the suggested solutions, it is possible to associate a first relative movement between two sensors to the closing/opening and a second and different relative movement between the same two sensors to the clamping/cutting force modulation.

**.** The relative movement between the two sensors for modulating the clamping/cutting force of the surgical instrument can be induced to be amplified with respect to the movement of a portion of the control interface on which the surgeon's fingers act directly, to promote precise and reliable detection.

**.** By virtue of the suggested solutions, the relative movement between two sensors according to a first relative movement direction is associated with opening and closing while the relative movement between the same two sensors according to a second relative movement direction is associated only with the clamping/cutting force modulation.

**.** The relative movement between two sensors according to a first relative movement direction associated with the opening/closing can be a first defined curved trajectory, while the relative movement between the same two sensors according to a second relative movement direction associated with the force modulation only can be a second and different defined curved trajectory, in which for example said second and different defined curved trajectory has an opposite concavity to said first defined curved trajectory and/or the instantaneous center of rotation is different for the first and second curved trajectory.

**.** The relative movement between the two sensors according to a first relative movement direction associated with opening and closing can be a variation of relative distance, while the relative movement between the same two sensors according to a second relative movement direction associated with force modulation alone can be a variation of orientation.

**.** The second relative movement direction associated with the force modulation can only be moved upon reaching the end-of-stroke of the first relative movement trajectory associated with the closing/opening movement. Reaching the end-of-stroke of the first trajectory can be associated with the position of complete closing of the master gripper. Reaching the end-of-stroke is thus clearly perceived by the user during the actuation of said imposed control movement. The end-of-stroke can be obtained by the contact between two free ends associated with two sensorized portions of the master device, respectively, to be intuitive to an operator. The possibility of further relative movement according to said first movement direction can be inhibited upon reaching the end-of-stroke, while it can be allowed to move only according to the second movement direction. In the master device, a second, further end-of-stroke position associated with said second relative movement direction and the transfer of the maximum clamping/cutting force/torque can be included.

**.** It can be included that both said first relative movement direction and said second relative movement direction are obtainable by actuating a manipulative movement imposed by the user according to a single movement direction, for example a relative approaching movement of two interface parts for the user. The manipulation motion can lie on a defined curve and/or on a straight line and can be determined by counting the surgeon's fingers on respective manipulation portions of the master device.

**.** A first part of the manipulation movement can be associated with the actuation of said first relative movement direction and thus of closing/opening. A second part of the manipulation movement can be associated with the actuation of said second relative movement direction and thus of clamping/cutting force/torque modulation.

**.** The manipulative movement along said manipulation direction can be associated with the application of a first force to induce said first relative movement direction associated with opening and closing, and a second force to induce said second relative movement direction associated with only gripping/cutting force modulation of the slave surgical instrument. The first force can be constant. The second force can grow with the control movement until the second further end-of-stroke position is reached.

**.** The master device can consist of two parts (e.g., two rods) connected/hinged in a rotational and/or translational connection joint. The connection joint between the two parts can be elastically preloaded so as to keep the master device in a maximum open state. An opening end-of-stroke can be included. Each part can in turn consist of two rigid sections, one proximal and one distal, connected by an elastic means. The two parts can be separated and connected by an elastic means as an additional component, or the two parts are part of the same piece and the elastic means is an elastic hinge, for example made with a reduced-thickness section of said same piece, which can have a folded and/or curved sheet body.

**.** The proximal part can include portions or surface areas where the fingers rest (for example at the manipulation portions), while the distal part can include portions where tracking sensors can be inserted (for example at operating portions).

### . Brief description of the figures

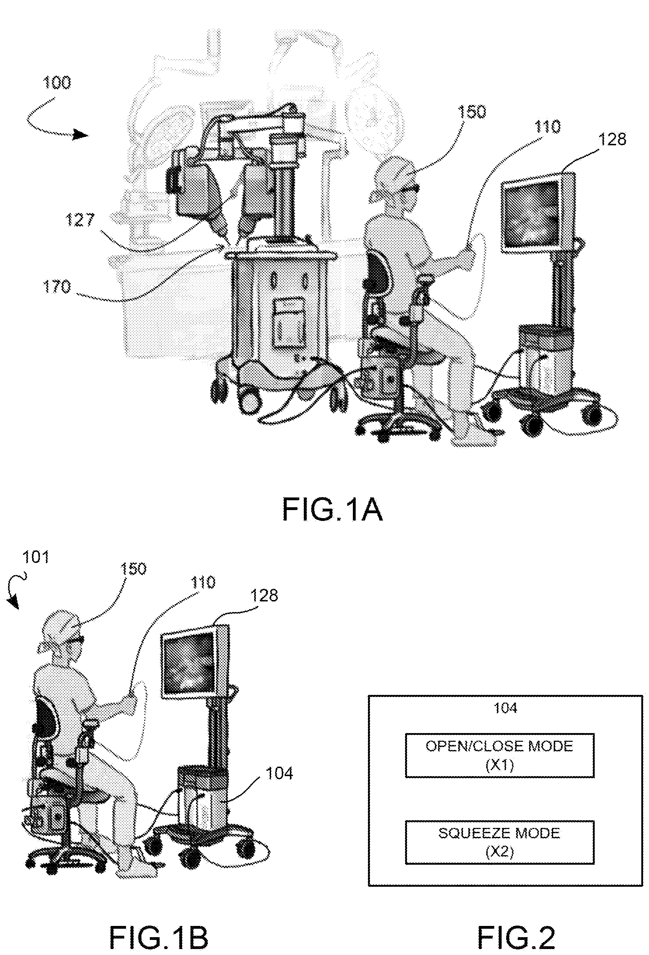

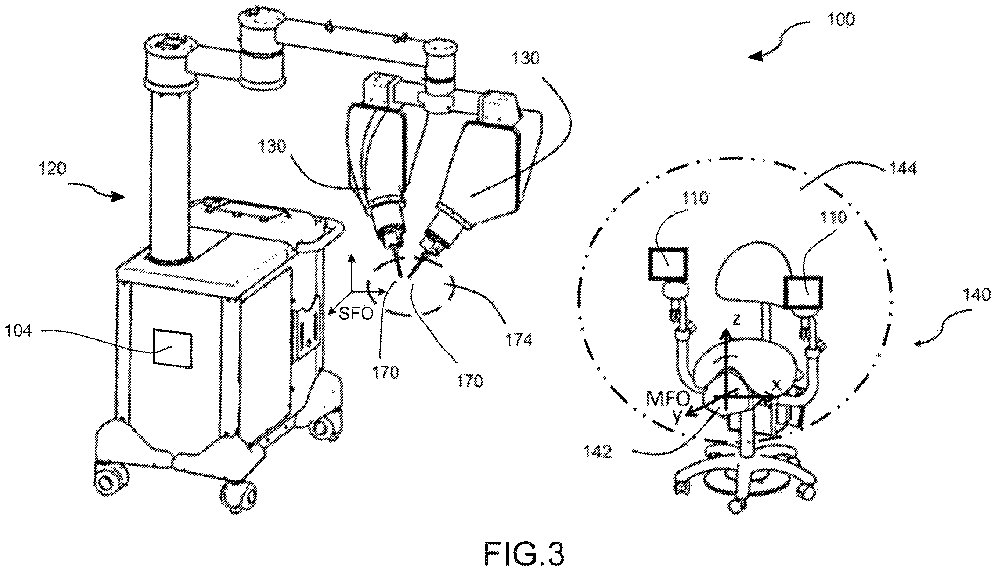

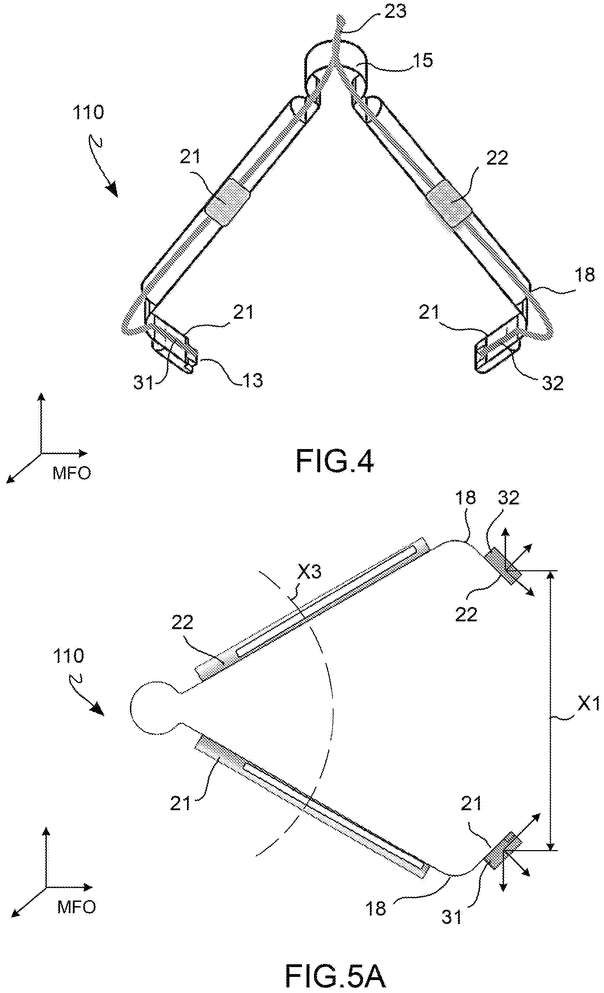

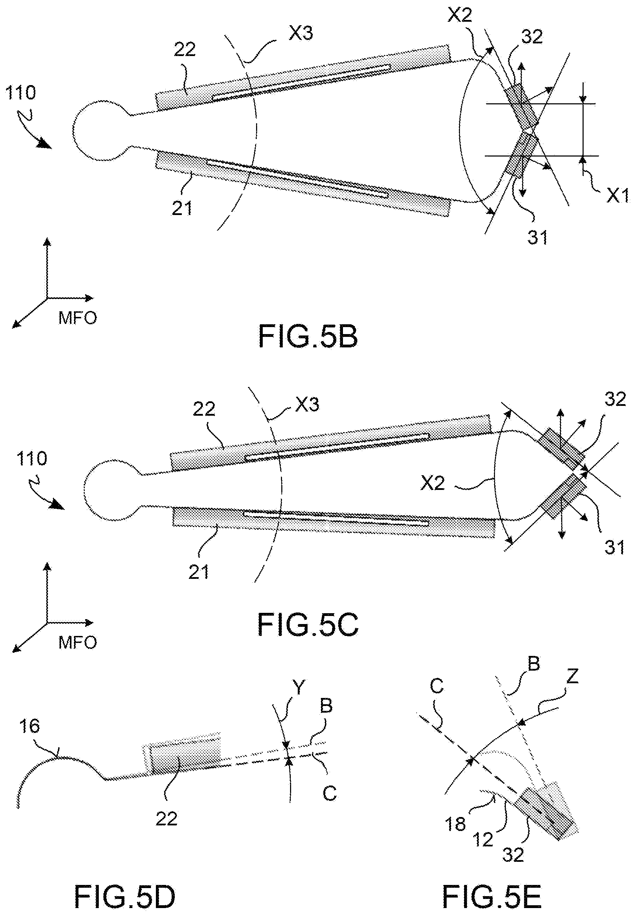

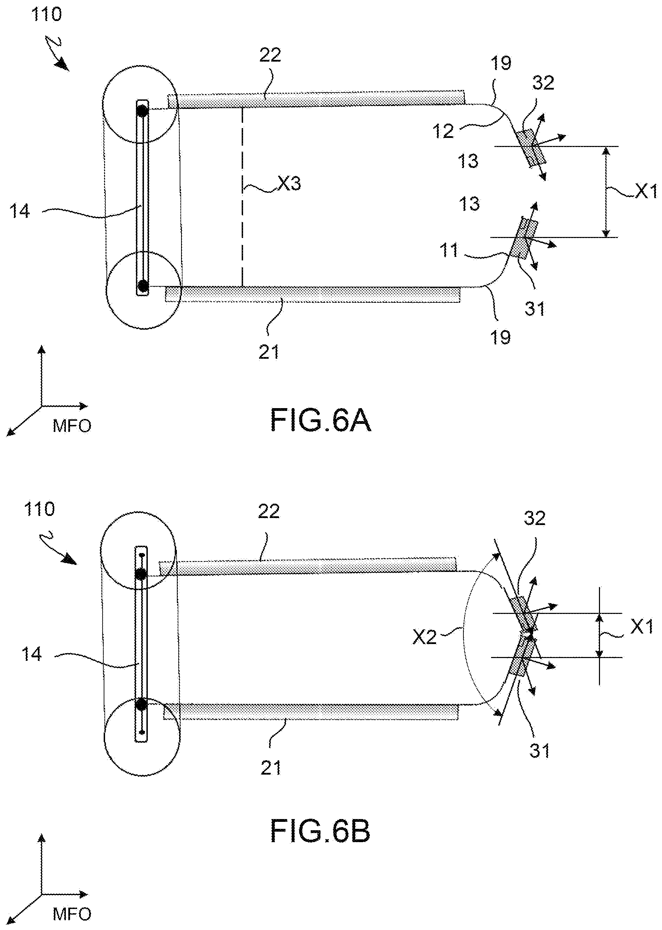

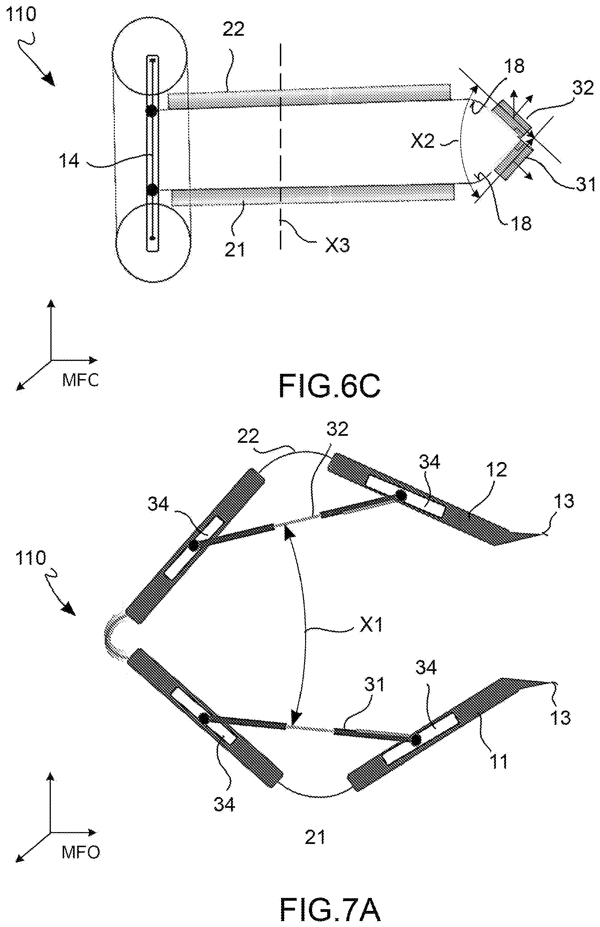

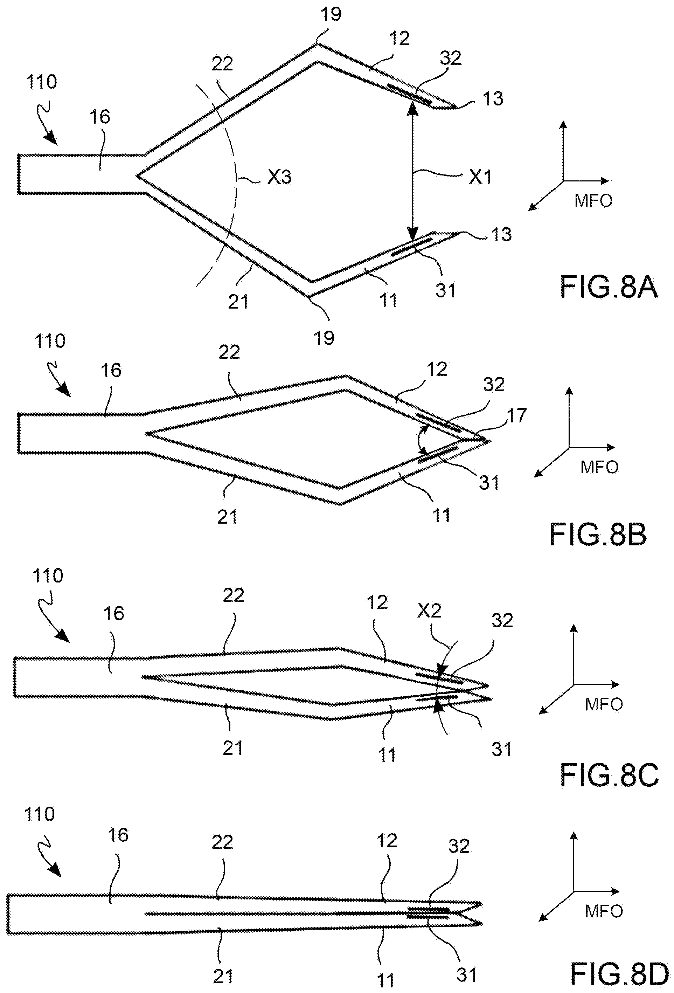

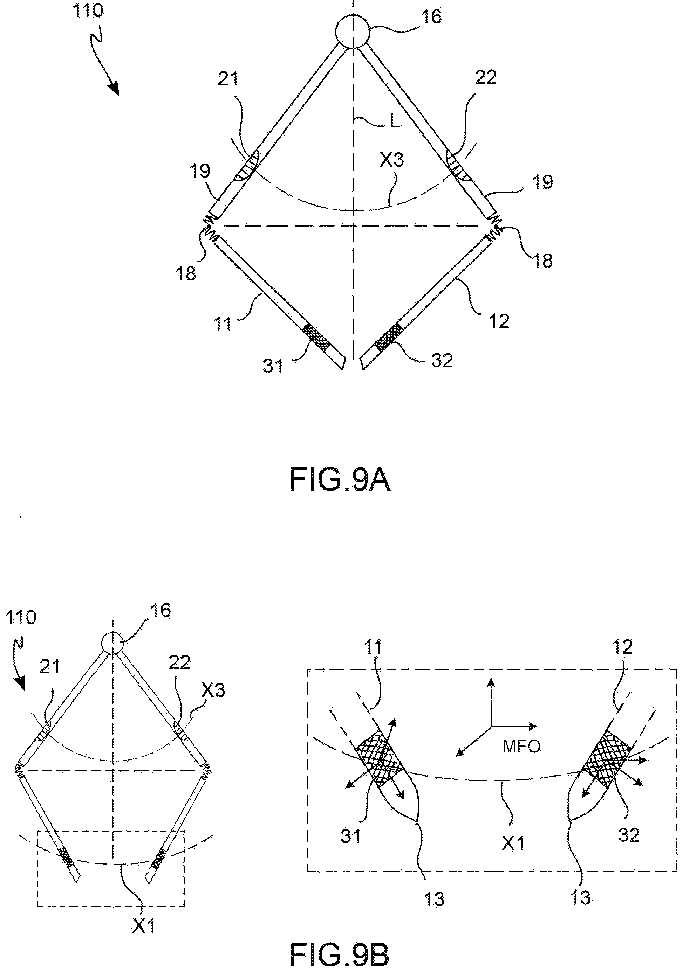

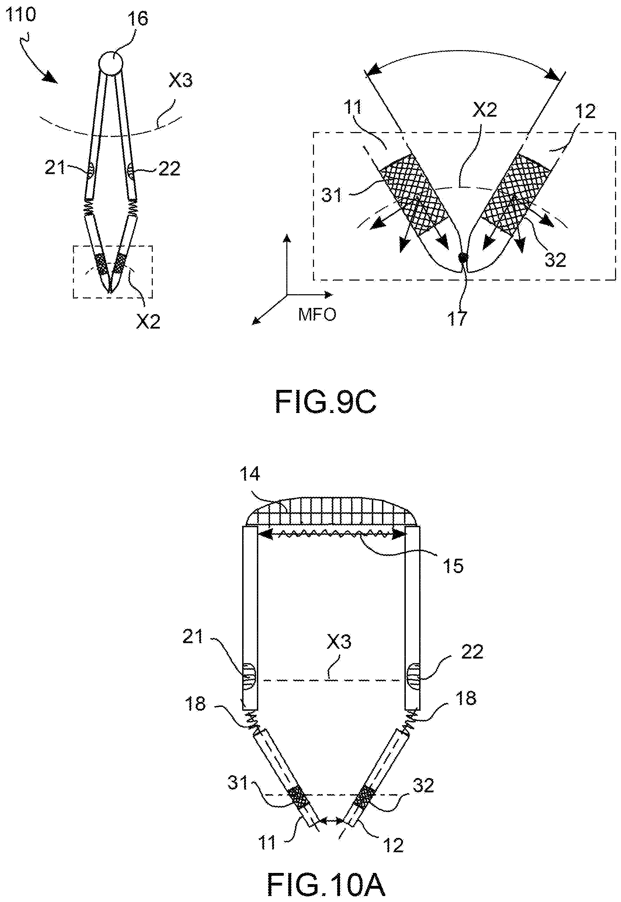

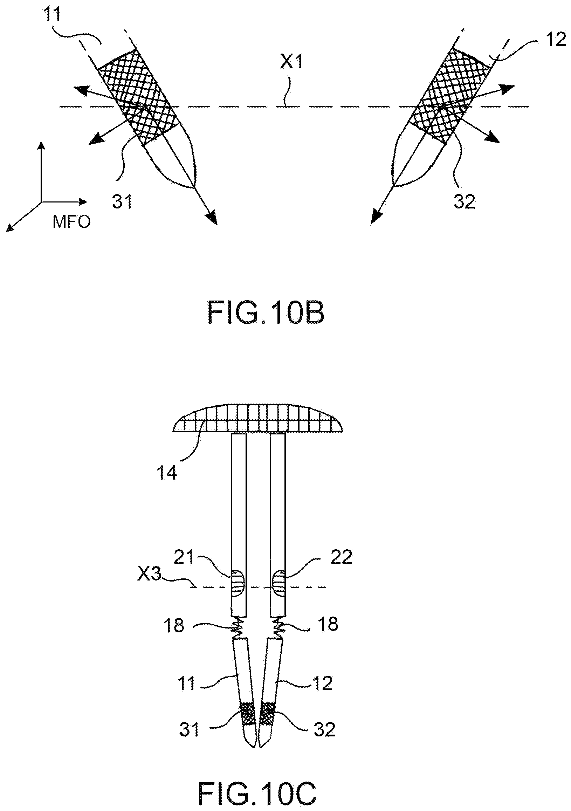

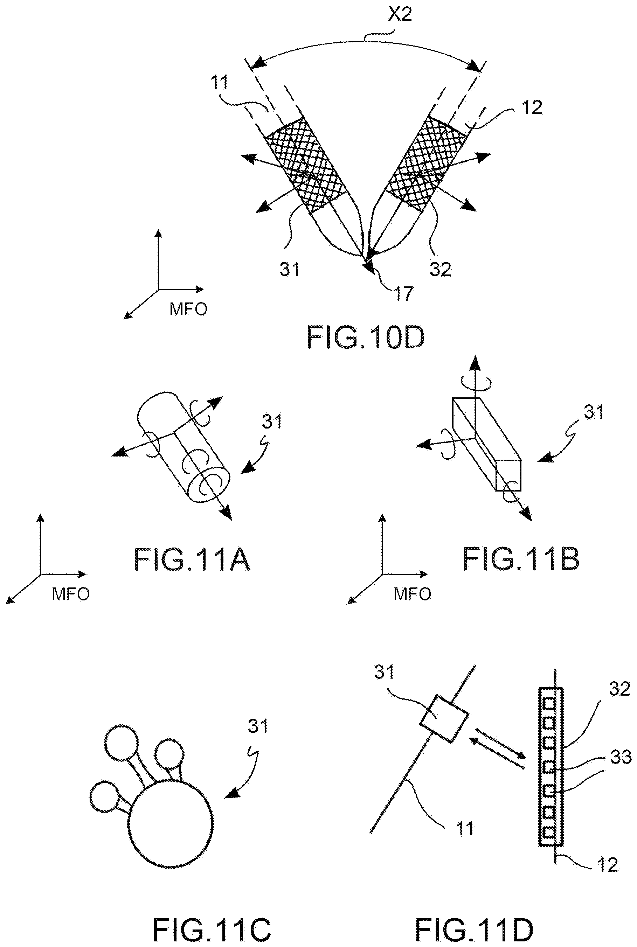

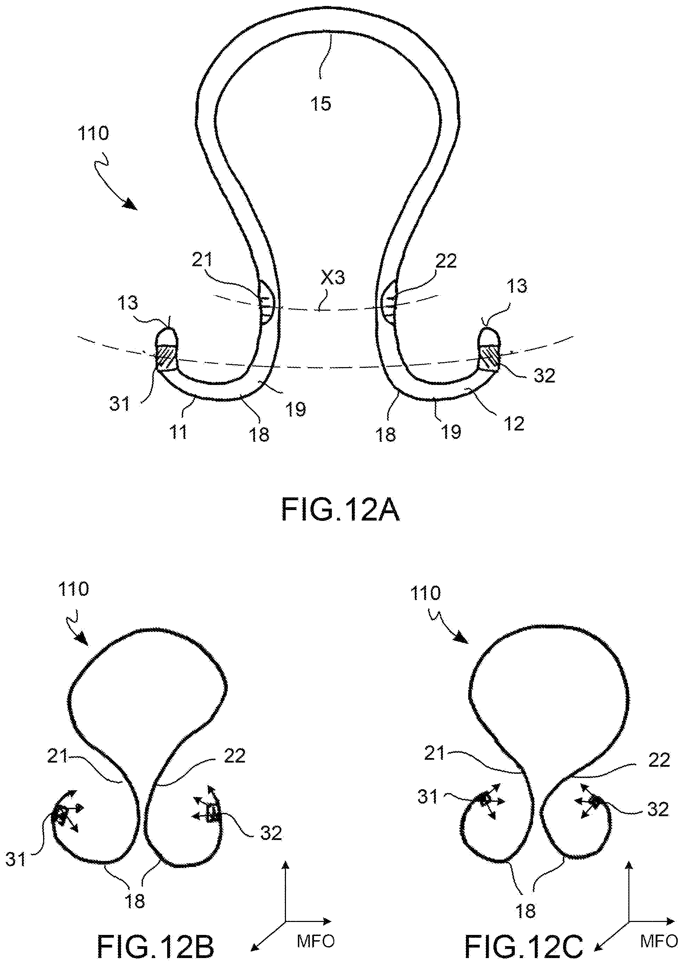

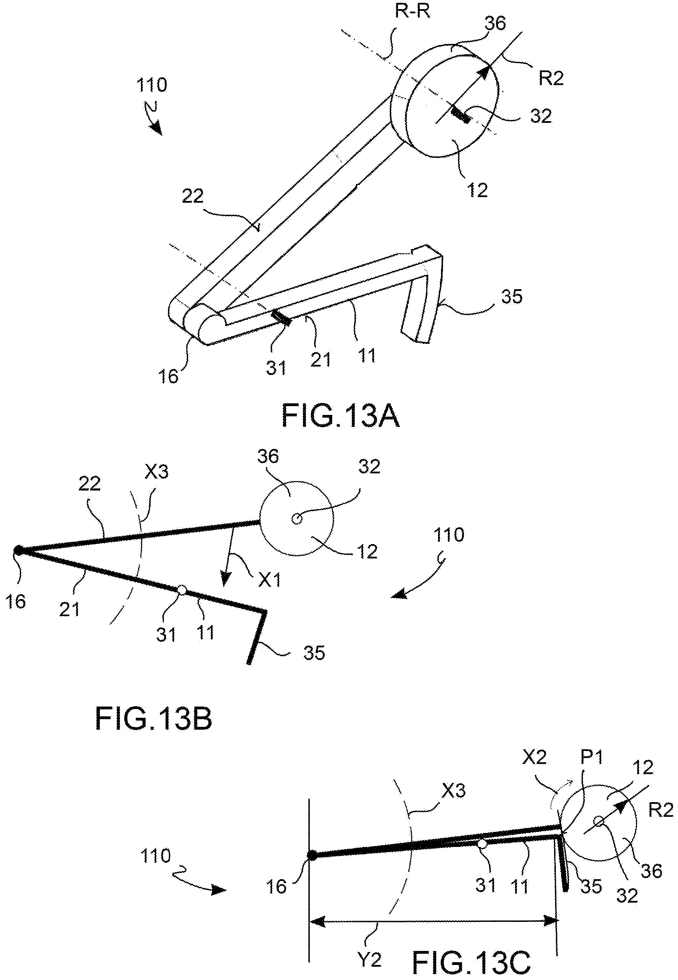

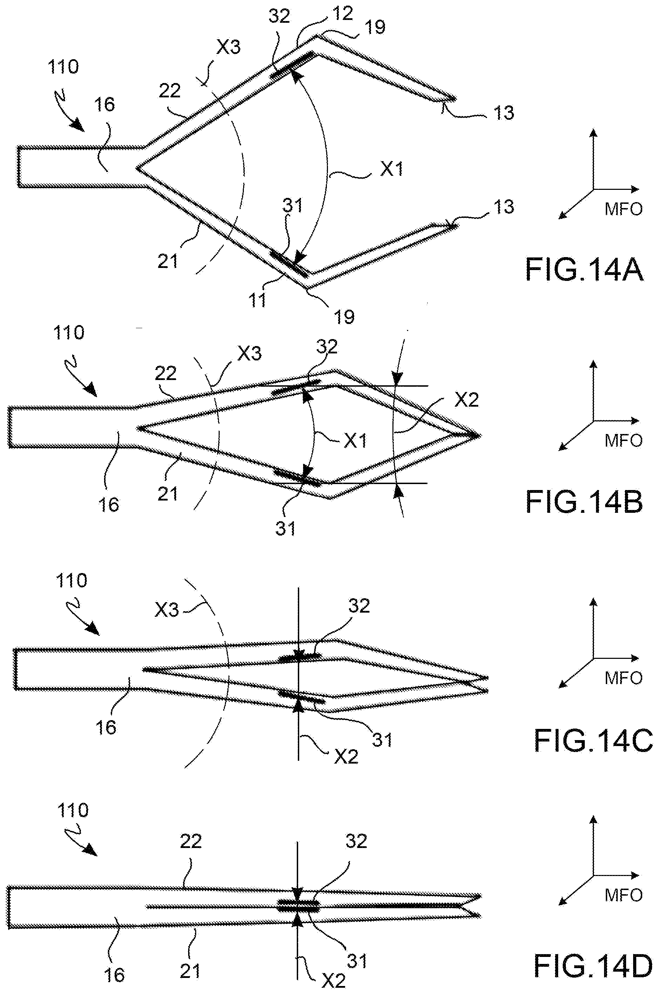

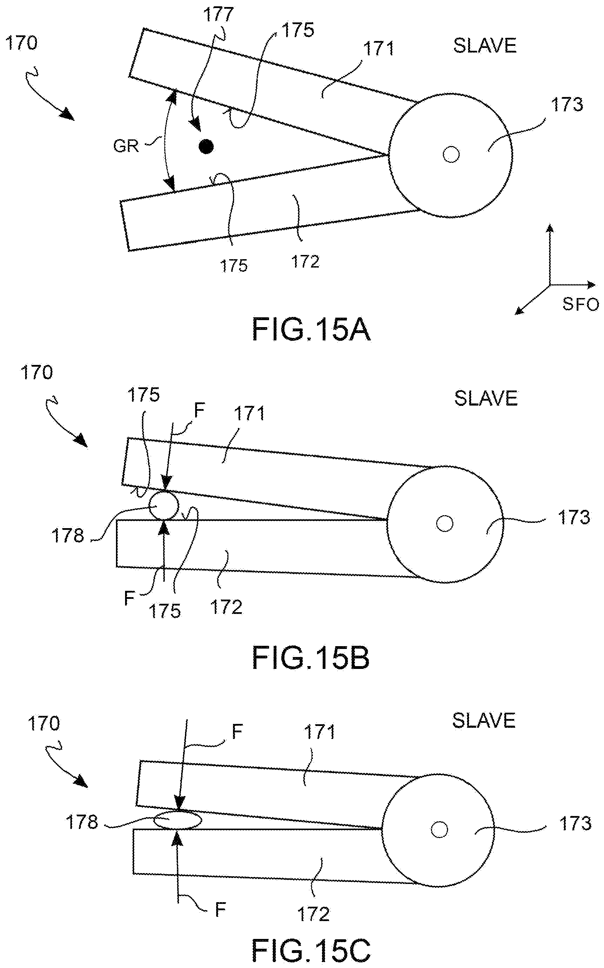

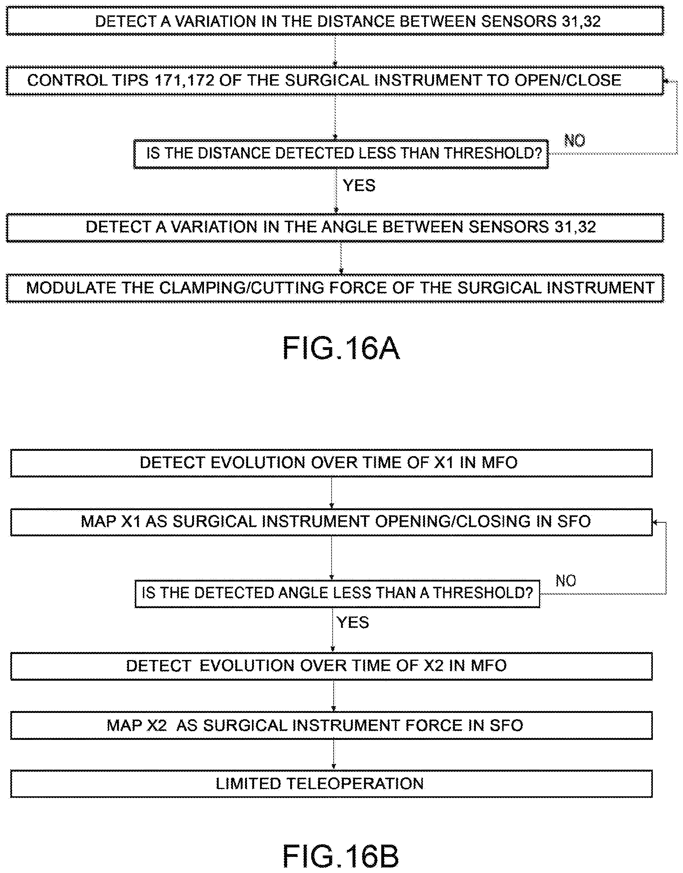

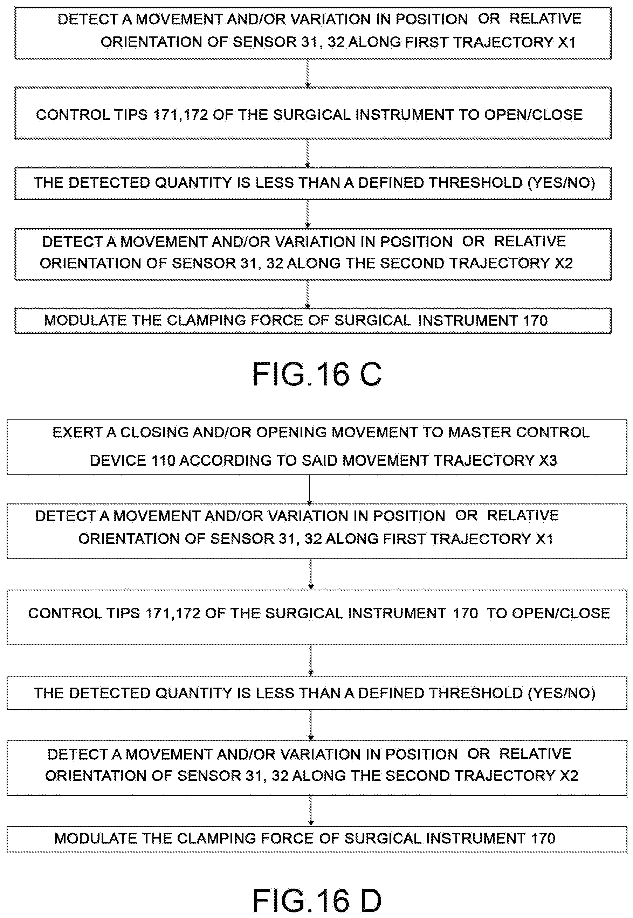

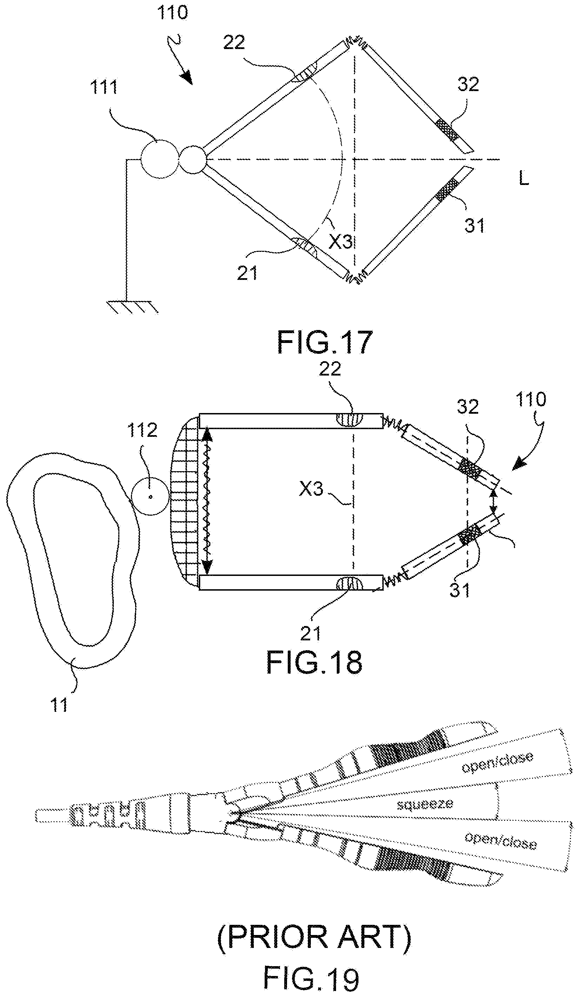

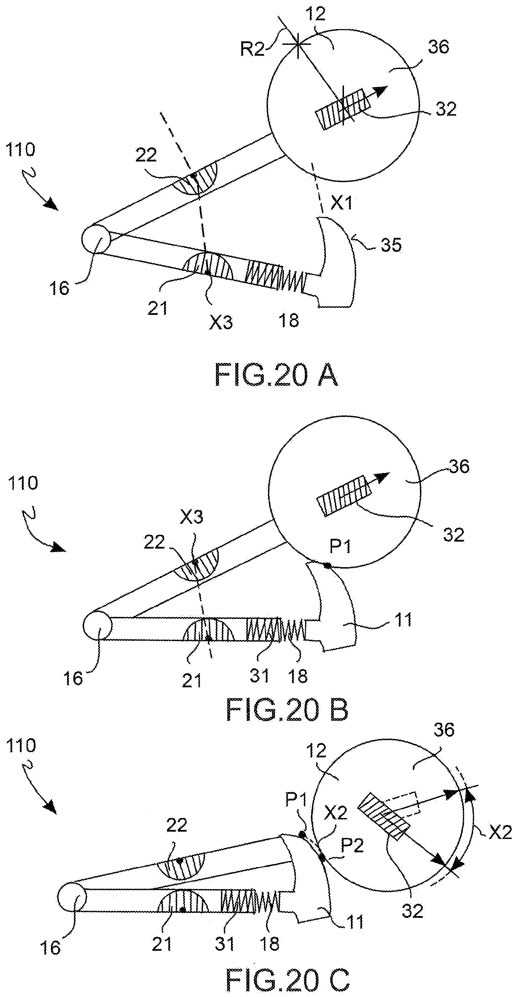

**.** Further features and advantages of the invention will become apparent from the following description of preferred embodiments, given by way of non-limiting indication, with reference to the accompanying drawings which are briefly described below. It should be noted that references to "an" embodiment in this disclosure do not necessarily refer to the same embodiment, and are to be understood as at least one. Moreover, for reasons of conciseness and reduction of the total number of figures, a certain figure can be used to illustrate the features of more than one embodiment, and not all the elements of the figure can be necessary for a certain embodiment.
**.** Figure 1 A is an axonometric view of a robotic system for medical or surgical teleoperation, according to an embodiment.
**.** Figure 1 B is an axonometric view of a system for simulating a medical or surgical teleoperation, according to an embodiment.
**.** Figure 2 is a diagram of a system control unit, according to an embodiment.
**.** Figure 3 is an axonometric view diagrammatically showing a robotic system for medical or surgical teleoperation, according to an embodiment.
**.** Figure 4 is an axonometric view of a master control device, according to an embodiment.
**.** Figures 5 A-C are plan views of the master control device in figure 4, in (A) open configuration, (B) first closed configuration, and (C) second closed configuration, respectively.
**.** Figures 5 E-D are diagrammatic views of transitions between the first and second closed configuration and resulting from overlaps between details of figures 5 A and 5 B.
**.** Figures 6 A-C are plan views of a master control device, according to an embodiment, shown in (A) open configuration, (B) first closed configuration, and (C) second closed configuration, respectively.
**.** Figures 7 A-C are plan views of a master control device, according to an embodiment, shown in (A) open configuration, (B) first closed configuration, and (C) second closed configuration, respectively.
**.** Figures 8 A-D are plan views of a master control device, according to an embodiment, shown in (A) open configuration, (B) first closed configuration, and (C-D) second closed configuration, respectively.
. Figure 9 A diagrammatically shows in plan view a master control device, according to an embodiment.
**.** Figure 9 B diagrammatically shows the master control device in Figure 9 A in open configuration, in which an enlargement of the portion in the box is shown.
**.** Figure 9 C diagrammatically shows the master control device in figure 9 A in closed configuration, in which an enlargement of the portion in the box is shown.
**.** Figure 10 A diagrammatically shows in plan view a master control device, according to an embodiment, shown in open configuration.
**.** Figure 10 B shows an enlarged view of a detail of figure 10 A.
**.** Figure 10 C diagrammatically shows the master device in figure 10 A in closed configuration.
**.** Figure 10 D shows an enlarged view of a detail of figure 10 C.
**.** Figures 11 A-C show in axonometric view a tracking sensor, according to some embodiments.
**.** Figure 11 D diagrammatically shows a pair of tracking sensors, according to an embodiment.
**.** Figures 12 A-C are plan views of a master control device, according to an embodiment, illustrated in (A) open configuration, (B) first closed configuration, and (C) second closed configuration, respectively.
**.** Figure 13 A shows in axonometric view a master control device, according to an embodiment.
**.** Figures 13 B-C diagrammatically show in plan view the master device in figure 13 A, shown in (B) open configuration and in (C) closed configuration, respectively.
**.** Figures 14 A-D are plan views of a master control device, according to an embodiment, shown in (A) open configuration, (B) first closed configuration, and (C-D) second closed configuration, respectively.
**.** Figures 15 A-C are diagrammatic views of a slave surgical instrument, according to an embodiment, illustrated in (A) open configuration, (B) grasping configuration, and (C) forcibly grasping configuration, respectively.
**.** Figures 16 A-D show block diagrams according to some embodiments of a control method.
**.** Figures 17 and 18 diagrammatically show a master control device, according to some embodiments.
**.** Figure 19 shows a known example of master control device.
**.** Figures 20 A-C are plan views of a master control device, according to an embodiment, illustrated in (A) open configuration, (B) first closed configuration, and (C) second closed configuration, respectively.

### . Detailed description of some embodiments

**.** Reference throughout this description to "an embodiment" means that a particular feature, structure or function described in relation to the embodiment is included in at least one embodiment of the present invention. Therefore, the formulation "in an embodiment" in various parts of this description do not necessarily all refer to the same embodiment. Moreover, particular features, structures or functions such as those shown in different drawings can be combined in any suitable manner in one or more embodiments.

**.** In accordance with a general embodiment, a robotic system 100 for medical or surgical teleoperation is provided.

**.** It is understood that the teachings of the present disclosure also apply in the case of a medical or surgical teleoperation simulation system 101.

**.** The robotic system 100 comprises at least one slave surgical instrument 170 provided with a degree of freedom of opening/closing GR, and at least one master control device 110 (or "gripper" 110, according to a widespread English term) for controlling at least the degree of freedom of opening/closing GR of the at least one slave surgical instrument 170.

**.** As shown for example in figure 2, the robotic system 100 (and/or the simulation system 101) further comprises at least one control unit 104. The control of the degree of freedom of opening/closing GR or grip GR of the slave surgical instrument 170 comprises two modes, a first mode (OPEN/CLOSE MODE) which concerns the control of the relative movement of the two tips 171, 172 relatively towards (closing) or away from (opening), and a second mode (SQUEEZE MODE, i.e., clamping or also meaning cutting) which relates to the modulation of the clamping/cutting force between the operating portions 175 of said two tips and which does not necessarily determine a relative movement between the two tips 171, 172. The master control device 110 of the robotic system 100 is configured to control both modes of the degree of freedom of opening/closing GR of the slave surgical instrument 170.

**.** As shown for example in figure 15-A, a slave surgical instrument 170 can comprise two tips 171, 172 or jaws 171, 172 constrained in a rotational joint 173 to rotate relatively about a common axis. Each tip or jaw can comprise an operating portion 175, such as a gripping surface and/or a blade and/or a counter-blade. For example, the slave surgical instrument 170 can be a gripper, dilator, needle-driver/sutures-cutter, surgical scissor, electro-cauterizing, and/or other type of instrument.

**.** The at least one slave surgical instrument 170 can belong to a slave robotic assembly 120 of the robotic teleoperation system. As shown for example in figure 3, the slave robotic assembly 120 can comprise, in addition to the at least one slave surgical instrument 170, at least one robotic manipulator 130 drivable under the control of the master control device 110 movable in the master workspace 144 to control the slave surgical instrument 170 in the slave workspace 174. A camera 127 or microscope 127 can be provided, for viewing the surgical site on a dedicated display 128.

**.** In accordance with a preferred embodiment, when in operating conditions, the robotic system 100 is configured to control the at least one slave device 170 based on the detection of position and/or orientation information of the at least one master device 110. For example, a control point 177 is defined with multiple degrees of freedom with respect to the slave global reference system SFO, the control point 177 being belonging to or rigidly associated with the slave surgical instrument 170, which can be located between the operating portions 175 of the tips 171, 172, and which is controlled based on the detection of position and/or orientation information of the master device 110 in the master workspace 144 with respect to the master global reference system MFO.

**.** To detect position and/or orientation information of the master device 110, a tracking field emitter 142, e.g., an electromagnetic field emitter, can be provided, which defines a tracking volume in which the master workspace 144 is obtained. The tracking of the master device 110 can be optical, and/or by means of infrared sensors, and/or by accelerometers, and/or by means of inertial platform (IMU), and/or any combination of the foregoing.

**.** Not necessarily the at least one master device 100 is a master device of the flying type i.e., kinematically unconstrained (groundless) to the operating console 140, although in accordance with a preferred embodiment, the at least one master device 100 is a master device of the flying type, i.e., unconstrained.

**.** The master control device 110 can be a master device of the type kinematically constrained to the operating console by means of a support 111 (for example: gymbal and/or motorized kinematic chain), as shown for example in figure 17, in which sensors 31, 32 are used to detect position and/or orientation information so as to control at least the degree of freedom of opening/closing GR of the slave surgical instrument 170, and in which for example orientation and global position information of the master device 110 can be controlled by means of the support 111 or constraint 111.

**.** The master control device 110 comprises at least two tracking sensors 31, 32 comprising a first tracking sensor 31 and a second tracking sensor 32. Of course, the term "tracking sensors 31, 32" is intended to indicate both electromagnetic sensors (figures 11 A-B) and optical sensors (markers, figure 11 C), as well as accelerometers, as well as inertial platforms, as well as infrared sensors, and any combination thereof. As shown for example in figure 11 D, a first sensor 31 can be provided, which is an infrared transmitter and a second sensor 32 which is a detector strip comprising a plurality of detectors 33 for example arranged longitudinally, so that from the detection of the time-of-flight of the return signal it provides information on the distance between the sensors 31, 32, while the location of the point or detector 33 irradiated on the detector strip provides information on the angle between the sensors 31, 32.

**.** Advantageously, the first tracking sensor 31 and the second tracking sensor 32 are associated, e.g., fixed, to portions of the master device 110 which are mutually movable under the action imparted by the surgeon's hand 150, respectively. The action imparted by the surgeon's hand 150 thus determines the variation of the geometric relationship between the two tracking sensors 31, 32, i.e., the modification, as a result of this manipulation action of the master control device 110.

**.** With further advantage, the control unit 104 of the robotic system 100 for medical or surgical teleoperation is configured to detect a first variation of the geometric relationship between the first tracking sensor 31 and the second tracking sensor 32 of the master control device 110 along a first direction X1, and to move in OPEN/CLOSE MODE a slave surgical instrument 170, based on the first variation of the detected geometric relationship. The detection of a movement between the two tracking sensors 31, 32 along the first direction X1 is thus interpreted by the system control unit as a command aimed at moving the slave surgical instrument 170 to open or close, for example moving the tips 171, 172 or jaws 171, 172 of the slave surgical instrument away from or toward each other.

**.** With further advantage, the control unit 104 of the robotic system 100 for medical or surgical teleoperation is configured to detect a second variation of the geometric relationship between the first tracking sensor 31 and the second tracking sensor 32 of the master control device 110 along a second direction X2, and vary (SQUEEZE MODE) the clamping/cutting force/torque of the slave surgical instrument 170, based on the second variation of the detected geometric relationship. The detection of a movement between the two tracking sensors 31, 32 along the second direction X2 is thus interpreted by the system control unit as a command aimed at modulating the closing/gripping/cutting (or opening) force of the slave surgical instrument 170, for example by clamping the operating portions 175 of the tips 171, 172 or jaws 171, 172 of the slave surgical instrument against each other.

**.** Such a system is thus capable of recognizing and discriminating an opening/closing movement command of the slave degree of freedom of opening/closing GR from a force modulation command applied by the slave surgical instrument 170, based on the relative movement direction X1, X2 between two sensors 31, 32 operatively associated with the body of the master device 110.

**.** In particular, the relative movement between the two sensors according to a first relative movement direction X1 can be associated with the opening and closing of the slave while the relative movement between the same two sensors according to a second different relative movement direction X2 can be associated with the gripping force modulation along of the slave.

**.** In a preferred embodiment both the first relative movement direction according to a trajectory or relationship X1 and the second relative movement direction according to a trajectory or relationship X2 between the two sensors are both obtainable by actuating a control movement imposed by the user according to a single direction of movement or trajectory X3.

**.** Said trajectory X3 can be identified by the special manipulation portions 21 and 22 and by the relative movement thereof exertable in these points directly from the user's fingertips.

**.** The relative position of the surface areas where the fingers rest on the master 21, 22 can define said single movement direction X3.

**.** In a preferred embodiment said control movement lies on a defined curve or arc.

**.** In a different embodiment said control movement lies on a straight line.

**.** A first part of the control movement can be associated with the actuation of said first relative movement direction and thus of closing/opening.

**.** A second part of the control movement can be associated with the actuation of said second relative movement direction and thus of gripping force modulation.

**.** The position and/or orientation information detected by the tracking sensors 31, 32 can also be used to control the position and orientation (TRACKING MODE) of the slave surgical instrument 170, e.g., the control point 177.

**.** The translations of the control point 177 of the slave surgical instrument 170 can be prevented or inhibited by the system control unit 104 during a clamping/cutting force modulation (SQUEEZE MODE) condition characterized by the relative movement X2 of the two sensors.

**.** The system can store the expected displacement of the control point 177 due to the command of the master device during the force modulation mode (SQUEEZE MODE) and in particular to the deformation of the master device body.

**.** The possibility of further relative movement according to said first movement direction X1 can be mechanically inhibited upon reaching the end-of-stroke P1, while it can be possible to move only said second relative movement direction X2

**.** The midpoint calculated with the distance or with the relative angle between the sensors can be kept mechanically unchanged and constant during the entire actuation of said second relative movement direction associated with the force modulation.

**.** The slave control point 177 associated with the position of the master can be that it does not move during the actuation and control of the only degree of closing/opening and force modulation

**.** The midpoint calculated with the distance or with the relative angle between the sensors can be kept unchanged and constant during the entire actuation of said second relative movement direction associated with the force modulation.

**.** In accordance with a preferred embodiment, the control unit 104 of the robotic teleoperation system is configured to detect a variation in distance X1 between the first tracking sensor 31 and the second tracking sensor 32, and move the slave surgical instrument 170 to open/close, based on the detected variation in distance X1. In accordance with this embodiment, the control unit 104 is further configured to detect a relative orientation variation X2 between the first tracking sensor 31 and the second tracking sensor 32, and based on the relative orientation variation vary the closing/gripping/cutting force/torque of the slave surgical instrument 170.

**.** The relative movement between two sensors according to a first relative movement direction X1 associated with opening and closing can be a first defined curved trajectory while the relative movement between the same two sensors according to a second relative movement direction X1 associated with force modulation alone is a second and different defined curved trajectory

**.** In an embodiment, the second and different defined curved trajectory has a concavity opposite said first defined curved trajectory.

**.** In an embodiment the relative movement between two sensors according to a first relative movement direction associated with opening and closing is a variation of relative distance while the relative movement between the same two sensors according to a second relative movement direction associated with force modulation alone is a variation of orientation.

**.** In an embodiment the relative movement between two sensors according to a first relative movement direction associated with opening and closing is a variation of orientation described by a first geometrical mathematical relationship and the relative movement between the same two sensors according to a second relative movement direction associated with force modulation only is a second variation of orientation described by a second different geometrical mathematical relationship.

**.** The master control device 110 adapted to control a degree of freedom of opening/closing GR of a slave surgical instrument 170 can take various shapes.

**.** In accordance with a preferred embodiment, the master control device 110 is a master device of the kinematically unconstrained type to any element of the operating console 140, i.e., it is of the flying or groundless type.

**.** The master device 110 comprises a body having a first operating portion 11 and a second operating portion 12, which are mutually movable under the action imparted by the hand of a surgeon 150.

**.** The surgeon 150 is not necessarily in contact with the operating portions 11, 12, which for example can be at a certain longitudinal distance from other special manipulation portions 21, 22 of the master device 110 which are precisely intended to be directly manipulated by the fingers of the surgeon 150 when in operating conditions.

**.** The master control device 110 further comprises a first tracking sensor 31 in operating connection with the first operating portion 11 of the body of the master device 110 and a second tracking sensor 32 in operating connection with the second operating portion 11 of the body of the master device 110. For example, the tracking sensors 31, 32 can be fixed, even removably, to the respective operating portions 11, 12 of the body of the master device 110.

**.** The body of the master device 110 does not necessarily comprise in a single piece the first operating portion 11 and the second operating portion 12, although in accordance with an embodiment the master device 110 comprises in a single piece the first operating portion 11 and the second operating portion 12.

**.** In accordance with an embodiment, the first operating portion 11 and the second operating portion 12 of the master device 110 are made in a separate piece and are assembled together.

**.** As shown for example in figure 4, the tracking sensors 31, 32 can comprise a data connection 23 to the control unit 104.

**.** In accordance with a preferred embodiment, the first operating portion 11 and the second operating portion 12 of the master device 110 are mutually movable under the action imparted by the hand of a surgeon 150 to take at least an open configuration, in which the first operating portion and the second operating portion are spaced apart by a distance X1, as shown for example in figure 5-A.

**.** Said at least one open configuration between the first operating portion 11 and the second operating portion 12 is preferably adapted to control, by means of the control unit 104, at least one enslaved open configuration of the slave surgical instrument 170, for example in which the tips 171, 172 form an opening angle therebetween which is greater than a certain threshold.

**.** The system can be configured to allow transitions between a plurality of open configurations of the master device 110 to which enslaved open configurations of the slave surgical instrument 170 correspond.

**.** In accordance with an embodiment, when in operating conditions, the opening angle between the tips 171, 172 of the slave surgical instrument 170 in the slave workspace 174 follows, in phase relation, the detected distance between the operating portions 11, 12 of the master device 110 in the master workspace 144. In other words, to control the transitions between the enslaved open configurations of the slave surgical instrument 170, the control unit 104 can be configured to detect information on the variation in distance X1 between the sensors 31, 32 and transmit control signals to the slave surgical instrument 170 aimed at moving the tips 171, 172 or jaws 171, 172 to open/close.

**.** In accordance with a preferred embodiment, the first operating portion 11 and the second operating portion 12 of the master device 110 are mutually movable under the action imparted by the hand of a surgeon 150 to take at least a closed configuration, in which the first operating portion 11 and the second operating portion 12 are each in contact with a respective abutment surface (for example formed by the other operating portion or by a third element for example extending between the operating portions 11, 12). At least one contact point 17 is thus defined when in the closed configuration.

**.** Said at least one closed configuration of the master device 110 is preferably adapted to control at least one enslaved closed configuration of the slave surgical instrument 170, for example in which the operating portions 175 of the tips 171, 172 of the slave surgical instrument 170 are in contact with each other or in contact with a body to be grasped 178.

**.** The second relative movement direction X2 associated with the force modulation can only be moved upon reaching the end-of-stroke P1 of the first relative movement trajectory X1 associated with the closing/opening movement or also said contact point 17.

**.** Upon reaching the end-of-stroke of the first trajectory P1 or contact point 17, the position of complete closing of the gripper can be associated.

**.** The robotic system can be configured to allow transitions between a plurality of closed configurations of the master device 110 to which enslaved closed configurations of the slave surgical instrument 170 correspond. Preferably, the at least one closed configuration of the master device 110 comprises a first closed configuration, as shown for example in figure 5-B, and a second closed configuration, as shown for example in figure 5-C.

**.** A transition between the first closed configuration and the second closed configuration can be adapted to control a variation in the closing/cutting/gripping force of the slave surgical instrument 170. In other words, the modulation of the closing force exerted by the operating portions 175 of the tips 171, 172 or jaws of the slave surgical instrument 170 is controlled by the transition between the first and the second closed configuration of the master device 110. The body of the master device 110 is designed so that a transition between the at least one open configuration and the first closed configuration results in a first variation of the geometric relationship between the first tracking sensor 31 and the second tracking sensor 32 and so that a transition between the first closed configuration and the second closed configuration results in a second variation of the geometric relationship between the first tracking sensor 31 and the second tracking sensor 32.

**.** Said first variation of the geometric relationship and said second variation of the geometric relationship occur along two different directions X1, X2.

**.** When the end-of-stroke P1 is reached, the two parts of the master are in contact at said contact point 17 and such a contact can be perceived by the user during the actuation of the imposed control movement X3 because it is characterized by greater rigidity.

**.** There can be an end-of-stroke position P2 associated with said second relative movement direction X2 and the transfer of the maximum grip force.

**.** The control movement along said control direction is associated with the application of a force F1 for inducing said first relative movement direction associated with opening and closing and a force F2 for inducing said second relative movement direction associated with force modulation only.

**.** In a constructive form F1 is constant.

**.** In a constructive form F2 grows with the control movement until the end-of-stroke position P2 is reached.

**.** In accordance with a preferred embodiment, the body of the master device 110 further comprises a first manipulation portion 21 and a second manipulation portion 22 for the action imparted by the surgeon's hand 150, in which the first manipulation portion 21 is in operating connection with the first operating portion 11 of the master device body and the second manipulation portion 22 is in operating connection with the second operating portion 12 of the master device body. When in operating conditions, the surgeon 150 grasps the master device 110 so as to manipulate, with his own fingers, for example, the manipulation portions 21, 22 of the body of the master device 110 which through the body of the master device itself determine a variation of the geometric relationship between the respective tracking sensors 31, 32. Between a manipulation portion 21, 22 and the respective operating portion 11, 12, the body of the master device 110 can comprise various means for transmitting an action imparted by the surgeon 150, such as: elastic devices 18, curved/bent sections 19, and others.

**.** In accordance with a preferred embodiment, the master device 110 further comprises an elastic device 15 between the first manipulation portion 21 and the second manipulation portion 22 of the body of the master device 110. The elastic device 15 is preferably configured to influence the first manipulation portion 21 and the second manipulation portion 22 in mutual distancing. The surgeon 150 thus moves the manipulation portions 21, 22 in relative approach, counteracting the bias of the elastic device 15. The elastic device 15 can be made in a single piece with the body of the master device 110 and for example is formed by an elastically deformable section of the body of the master device 110. The elastic device 15 can comprise a helical axial spring and/or a torsional spring.

**.** In accordance with a preferred embodiment both the first relative movement direction according to a trajectory or relationship X1 and a second relative movement direction according to a trajectory or relationship X2 between the two sensors are both obtainable by actuating a control movement imposed by the user according to a single direction of movement or trajectory X3.

**.** In particular, the first manipulation portion 21 and the second manipulation portion 22 are both movable at different and distinct times and portions by exerting a single movement direction X3 for example a relative approach/distancing or opening/closing direction of the manipulation points of the user.

**.** . The third direction X3 can be a straight translation direction and/or a circumferential arc, and/or a rotational direction.

**.** In accordance with an embodiment, the first manipulation portion 21 and the second manipulation portion 22 are forced to move with respect to each other along a single third direction, for example of relative approach/distancing. For example, a guide 14 and/or a rotational constraint 16 can be provided to constrain the relative movement between the manipulation portions 21, 22.

**.** In accordance with an embodiment, the first manipulation portion 21 and the second manipulation portion 22 are movable with respect to each other along a manipulation stroke having a certain magnitude of manipulation stroke, in which the first variation of the geometric relationship between the first tracking sensor 31 and the second tracking sensor 32 defines a first tracking stroke having a certain magnitude of first tracking stroke, and in which the second variation of the geometric relationship between the first tracking sensor 31 and the second tracking sensor 32 defines a second tracking stroke having a certain magnitude of second tracking stroke. Preferably, the sum of the first tracking stroke and the second tracking stroke is greater than the manipulation stroke.

**.** The resolution of the robotic teleoperation system in controlling the degree of freedom of opening/closing and the modulation of the gripping/cutting/closing force is thus improved because the magnitude of the relative movement of the operating portions 11, 12 provided with sensors 31, 32 is wider than the magnitude of the relative movement of the manipulation portions 21, 22. For example, the angle variation between the manipulation portions 21, 22 of the master device 110 between the open configuration and the second closed configuration is of a certain angular amount which is smaller than the corresponding angle variation between the operating portions 11, 12. In other words, such a master device 110 allows mechanically amplifying the magnitude of the control imparted by the surgeon in terms of opening/closing and modulation of the gripping/closing/cutting force, unlike the known master device solutions (figure 19).

**.** This can be obtained by selectively detecting different ways of varying the geometric relationship between the tracking sensors 31, 32.

**.** In accordance with a preferred embodiment, the master device 110 comprises between one manipulation portion 21, 22 and the respective operating portion 11, 12 a curved section 19 which describes a change of direction and is preferably made flexible. In other words, the body of the master device comprises a flexible portion describing a change of direction between the manipulation portion and the respective operating portion.

**.** Said curved section 19 describing a change of direction can have an elastic return, and/or can be a folded section, and/or can describe a broken line and/or can be a section describing a curved arc.

**.** In accordance with a preferred embodiment, the two different directions X1, X2 are distance between the sensors and angle between the sensors, respectively.

**.** As shown for example in figure 5-A, in which an open configuration of the master device 110 is shown, the robotic system 100 can be configured to control the degree of opening/closing GR of the slave surgical instrument 170 based on the distance X1 (i.e., the first direction X1) detected between the two tracking sensors 31, 32. Not necessarily the operating portions 11, 12 of the master device 110 each comprise a free end 13, although in accordance with a preferred embodiment each of the operating portions 11, 12 comprises a free end 13. The distance between the sensors 31, 32 can be a measure of the distance between the free ends 13 of the first operating portion 11 and the second operating portion 13.

**.** As shown for example in figures 5-B and 5-C, as the distance X1 (i.e., the first direction X1) between the tracking sensors 31, 32 decreases, the robotic system 100 can be configured to bring the slave surgical instrument 170 into a closed configuration, in which the clamping/closing/gripping/cutting force is in phase relationship with the angle X2 (i.e., the second direction X2) between the orientations between the tracking sensors 31, 32.

**.** Alternatively, the system can be configured so that the first direction X1 is associated with a detection of the angle variation between the orientations of the tracking sensors 31, 32 and the second direction X2 is associated with a detection of the distance variation between the tracking sensors 31, 32, for example distance variation along a longitudinal direction of the body of the master device 110.

**.** Of course, during the detection of the distance variation between the tracking sensors, said tracking sensors 31, 32 can also vary the relative orientation thereof.

**.** As shown for example in figures 5-D and 5-E, the angular stroke Y performed by the manipulation portion 22 of the master device 110 during a transition between the first closed configuration (dotted line indicated with the letter B) and the second closed configuration (dotted line indicated with the letter C) is much smaller than the angular stroke Z performed by the respective operating portion 12, i.e., by the sensor 32, to increase the resolution in the control of the modulation modes of the gripping/cutting force (SQUEEZE MODE). For example, the ratio of the angular strokes Y to Z can be 1 to 5, 1 to 10, or greater than 1 to 10, and for example the angular stroke Y is substantially 5° while the angular stroke Z is substantially 45°, 60°, or 90°.

**.** As shown for example in figures 6-A, 6-B and 6-C, the operating portions 11, 12 of the master device 110 can be constrained to translate with respect to each other, so as to keep the relative orientation between the two tracking sensors 31, 32 constant during a transition between the at least one open configuration and the first closed configuration, while during a transition between the first and the second closed configuration the relative orientation between the tracking sensors 31, 32 varies.

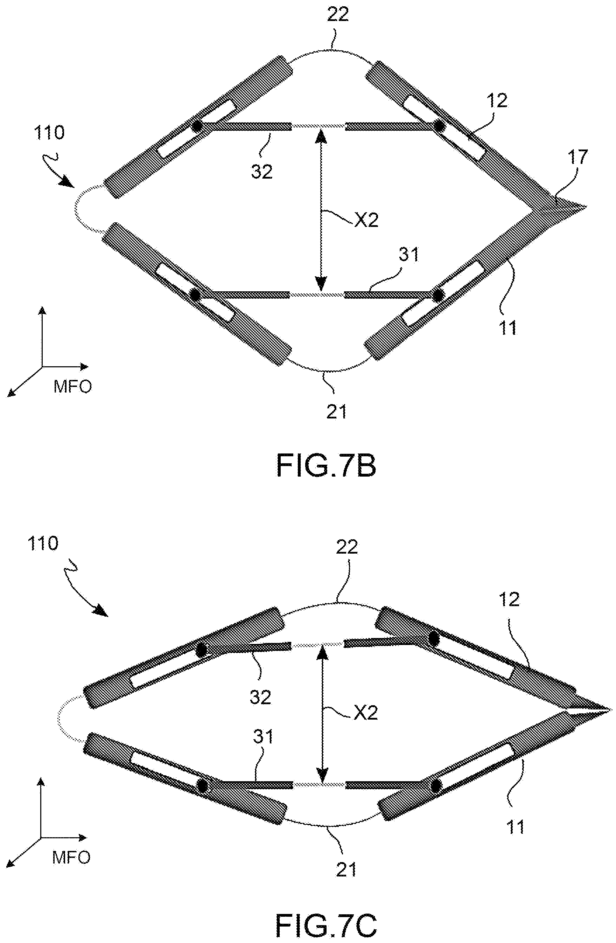

**.** As shown for example in figures 7-A, 7-B and 7-C, the tracking sensors 31, 32 can have rigid bodies (e.g., they can be mounted on rigid rods). In this example guides 34 are shown for the longitudinal sliding of the rigid body sensors 31, 32 and the respective operating portions 11, 12. Two first (or second) operating portions 11 can be provided, with a first (or second) manipulation portion 21 therebetween.

**.** The operating portions 11, 12 of the master device 110 can have constrained ends, to form a closed figure for example of the articulated parallelogram type.

**.** As shown for example in figures 8-A, 8-B, 8-C and 8-D, the manipulation portions 21, 22 can travel a relative approach/distancing path along a third curved direction X3 defining: a variation of the distance X1 between the sensors 31, 32 when the operating portions 11, 12 are in the open configuration, and a variation of the angle between the orientations of the two sensors 31, 32 when the operating portions 11, 12 are in the closed configuration.

**.** As shown for example in figures 9-A, 9-B and 9-C, the first variation of the geometric relationship between the tracking sensors 31, 32 occurs along a first direction X1 which is a curved direction, and preferably a circumferential arc centered in the constraint 16 between the manipulation portions 21, 22 of the master device body 11, while the second variation of the geometric relationship between the tracking sensors 31, 32 occurs along a second direction X2 which is always a curved direction, but with concavity opposite the first curved direction X1. Preferably, the second curved direction X2 is a circumferential arc centered at the contact point 17 between the operating portions 11, 12 of the master device 110.

**.** The second variation of the geometric relationship between the sensors 31, 32 can be determined by the elastic deformation of said first operating portion 11 and/or of said second operating portion 12 of the body of the master device 110.

**.** For example, the first operating portion 11 and the second operating portion 12 can comprise elastic cuffs which mount said tracking sensors 31, 32 and which are adapted to elastically deform during the manipulation of the master device 110.

**.** The articulated connection joint between the two parts of the master can be elastically preloaded to maintain the master in a state of maximum opening and abutment at said end-of-stroke point.

**.** Each part can also consist of two rigid sections, a proximal and a distal one connected by an elastic means or an elastic hinge with reduced thickness.

**.** As shown for example in figures 10-A, 10-B and 10-C, the operating portions 11, 12 of the master device 110 can be constrained to translate with respect to each other, so as to keep the relative orientation between the two tracking sensors 31, 32 constant during a transition between the at least one open configuration and the first closed configuration, while during a transition between the first and the second closed configuration the relative orientation between the tracking sensors 31, 32 varies. A transverse guide 14 pre-loaded with an elastic device 15 can be provided to guide the movement between the two manipulation portions 21, 22 of the master device.

**.** The contact point 17 between the operating portions 11, 12 of the master device 110 can be determined by a relative sliding contact between the operating portions 11, 12.

**.** As shown for example in figures 11-A and 11-B, each of the tracking sensors 31, 32 can be capable of detecting six degrees of freedom, of which three degrees of freedom of orientation and three degrees of freedom of position with respect to a reference point of a master global reference system MFO.

**.** The master device 110 can comprise an elastic element 18 associated with at least one, and preferably both, of the operating portions 11, 12. In accordance with an embodiment, an elastic element 18 is provided between each manipulation portion 21, 22 and the respective operating portion 11, 12 provided with tracking sensor 31, 32.

**.** At least one of the tracking sensors 31, 32 can be a fiber optic sensor, for example a deformation sensor of the Bragg grating type.

**.** As shown for example in figures 12-A, 12-B and 12-C, the body of the master device 110 can comprise in a single piece both manipulation portions 21, 22 and both operating portions 11, 12, in which the body of the master device 110 comprises a plurality of elastically deformable portions 15, 18, of which a first elastically deformable portion 15 between the interface portions 21, 22 and a second elastically deformable portion 18 between each interface portion 21, 22 and the respective operating portion 11, 12 provided with sensor 31, 32 and/or belonging to each operating portion 11, 12.

**.** The first variation direction X1 of the geometric relationship between the tracking sensors 31, 32 can be an approaching direction while the second variation direction X2 of the geometric relationship between the tracking sensors 31, 32 can be a distancing direction between the sensors.

**.** As shown for example in figures 13-A, 13-B and 13-C, the second operating portion 12 of the master device 110 can comprise a rotating member 36 of radius R2, and the first operating portion 11 of the master device 110 can comprise an abutment and dragging surface 35, adapted to form a rolling contact with the rotating member 36 of the master device 110.

**.** The abutment and dragging surface 35 is preferably a curved and convex surface. The abutment and dragging surface 35 can comprise a toothed portion which engages with a respective toothed counter-portion of the rotating member.

**.** For example, a first closing portion of the master device before the contact between the rotating member and the dragging surface is defined as a trajectory X1 of relative movement between the sensors and a relationship which can be associated with the slave opening and closing.

**.** The detection of the second relative movement direction X2 and the relative relationship associated with the force modulation, can be the detection of the relative orientation between the sensors 31 and 32, in which a sensor 32 is placed on the rotating member 36. Preferably, the sensor 32 is placed on the rotation axis R-R of the rotating member 36. The sensor 32 can be placed on the edge of the rotating member near or at the contact point with the dragging surface 35 of the first relative portion 11.

**.** When in operating conditions along the second direction X2, the closing of the body of the master device 110 expresses the dragging in rotation of the rotating member and the transfer of a proportional force modulation to the slave.

**.** The radius R2 of the rotating member can advantageously be chosen to be less than the distance Y2 between the constraint 16 and the rotation axis R-R of the rotating member, allowing an increase in the angular stroke or travel of the sensor 32 associated therewith and gaining an adequate resolution for the force modulation.

**.** In an embodiment, the rotating member can be preloaded by a torsional spring in a defined position when not dragged by the abutment and dragging surface. For example, with a sensor axis aligned with the direction of the first operating portion.

**.** Other tracking sensors can be provided.

**.** The abutment and dragging surface 35 can be mounted on an elastic pre-loading element 18 which ensures an adequate friction force during contact with the rotating member and ensures the dragging along the entire stroke thereof from a first contact point P1 in which the long trajectory movement X1 is exhausted and the long trajectory movement X2 begins at a point P2 in which the long trajectory movement X2 is exhausted.

**.** The abutment and dragging surface 35 can be curved and convex. The rotating member 36 can comprise a rotation axis R-R which is fixed with respect to the first operating portion 11.

**.** As shown for example in figures 14-A, 14-B, 14-C and 14-D, the first variation direction X1 of the geometric relationship between the sensors 31, 32 is an angular direction and the second direction X2 of the geometric relationship between the sensors 31, 32 is a distance.

**.** As shown for example in figure 18, the master control device 110 can be of the unconstrained type and comprise in addition to the gripper portion for controlling the degree of freedom of opening/closing GR of the surgical instrument 170, a wearable element 113 (for example a bracelet or a ring) which can be connected to the body of the master device 110 by means of a joint 112 or articulation 112. Alternatively or in addition to the wearable element 113, a knob or handle can be provided.

**.** The master device 100 can comprise elements for improving ergonomics, for example rests designed specifically for the palm of the surgeon 150 gripping the master device, and the like.

**.** As shown for example in figures 20-A, 20-B and 20-C, the first operating portion 21 can comprise an abutment and dragging surface 35 extending on a cantilevered appendage extending from the first operating portion 21 toward the second operating portion 22 mounting a rotating member 36. An elastic element 18 can be provided between the tracking sensor 31 and the abutment and dragging surface 35 of the first operating portion 21.

**.** With reference to the foregoing description, a control method for a robotic system 100 for medical or surgical teleoperation will now be described.

**.** The control method is for controlling a degree of freedom of opening/closing GR of a slave surgical instrument 170 controlled by a master control device 110.

**.** The control method comprises the steps of detecting a variation of the geometric relationship between a first tracking sensor 31 and a second tracking sensor 32 of the master device 110 along a first direction X1 and moving a slave surgical instrument 170 in an opening/closing GR mode.

**.** Advantageously, the method further comprises the steps of detecting a variation of the geometric relationship between said first tracking sensor 31 and said second tracking sensor 32 of the master device along a second direction X2 and varying, i.e., modulating the clamping/cutting force/torque of the slave surgical instrument 170.

**.** The degree of freedom of opening/closing GR of the slave surgical instrument 170 follows the variation of the geometric relationship between the sensors 31, 32 along the first direction X1, while the modulation of the clamping/cutting force/torque of the same slave surgical instrument 170 follows the variation of the geometric relationship between the sensors 31, 32 along the second direction X2.

**.** The control method can comprise the further step of performing one or more checks on the detected information on the variation of the geometric relationship between the sensors 31, 32, and enabling control transitions between the OPEN/CLOSE MODE and the clamping cutting (SQUEEZE MODE) mode only if said one or more checks are successfully passed.

**.** In accordance with an embodiment, said one or more controls comprise control the that the detected value of the variation of the geometric relationship along the direction X1 belongs to a set of allowable values, for example predetermined or determined iteratively in real time.

**.** In accordance with an embodiment, said one or more controls comprise verification of the structural integrity of the master device. This verification can comprise verification of the existence of predefined mathematical relationships between the sensors 31, 32, for example imposed by the geometry of the body of the master device 110.

**.** The method can comprise, during the step of varying the clamping/cutting force, the step of entering a limited teleoperation state, in which a subset of the degrees of freedom is not transmitted to the slave surgical instrument 170 or to the control point 177. Preferably, during SQUEEZE MODE, translations of the control point 177 are inhibited to avoid undesirable repositioning of the slave device positioning kinematic chain which can comprise an articulated end and/or motorized robotic axes. For example, control point translations are only inhibited if belonging to a specific direction, for example the longitudinal direction L of the master device 110 in the master workspace and thus of the surgical instrument 170 in the slave workspace.

**.** With reference to the above, the directions X1 and X2 are different directions and, for example, can be: linear distance variation and angular distance variation, i.e., angle between the orientations, or vice versa; rotation with respect to a first center of rotation and rotation with respect to a second center of rotation, the first and second centers of rotation can define very different radii, distance variation along a first direction and distance variation along a second direction incident thereto and for example orthogonal.

**.** The control method is adapted to be implemented by means of a system for medical or surgical teleoperation 100 comprising at least one master control device 110 according to any of the previously described embodiments.

**.** Of course, the step of detecting a movement, as well as the step of detecting a pose, as well as the step of detecting a variation in the geometric relationship between the tracking sensors 31, 32 can comprise the steps of: detecting information on the position and orientation of each tracking sensor 31, 32; and processing the information processed by the sensors (e.g., calculating) to derive information on movement/pose/variation in the geometric relationship.

**.** In accordance with an embodiment, as shown for example in figure 16-C, the method comprises the following steps:
- detecting a movement and/or a variation in position or relative orientation of the sensors 31,32 along the first trajectory X1;
- controlling the Opening/Closing of the tips 171, 172 of the surgical instrument 170,
- if the detected quantity is less than a defined threshold, then:
   - detecting a movement and/or variations of position or relative orientation of the sensors 31,32 along the second trajectory X2.
   - modulating the clamping force of the surgical instrument 170.

**.** The method can further comprise the step of exerting a closing and/or opening movement to the master control device 110 according to said movement trajectory X3.

**.** The master device 110 can be configured to control an electrosurgical instrument, e.g., an active monopolar and/or bipolar surgical instrument. In this case, the SQUEEZE MODE can be implemented to manage the delivery intensity of electrical energy through the slave surgical instrument 170.

**.** In accordance with a general embodiment, a computer program is provided, which is configured to perform the steps of the control method, according to any of the embodiments described above.

**.** By virtue of the features described above, provided either separately or in combination, where applicable, it is possible to respond to the needs mentioned above, and to obtain the listed advantages, in particular:
- it allows decoupling, on the master side, the opening/closing command from the clamping/cutting force modulation command;
- therefore, a better detection resolution of said commands given by the surgeon on the master device is allowed;
- in addition, a simpler and more precise control of critical variables (position/orientation of tracking sensors) is allowed, which can result in faster and more efficient data processing, and for these reasons provide a safer solution with respect to known examples of surgical or microsurgical teleoperation systems;
- a master control device of the flywheel type, or kinematically unconstrained to any element of the operating setup is provided, with improved performance with respect to known solutions, in particular with regard to the control of the opening/closing and the clamping/cutting force of the surgical instrument;
- the same tracking sensors (for example two tracking sensors) can also be used to control the position and orientation of the slave surgical instrument, which can comprise an articulated end actuated by means of actuation cables, and which for example can be provided with pitch, yaw and/or roll rotational joints, as well as can include a snake-type positioning mechanism comprising a plurality of stacked vertebrae;
- moreover, in the case of microsurgical teleoperation, the surgeon can consider it desirable to control the opening/closing and therefore the gripping/cutting of the slave device by means of minimal movements of his fingers, similar to what occurs in traditional microsurgical practice which is not assisted by robotic systems; therefore, by virtue of the present invention it is possible to meet this need, providing an increased resolution of detection on the master side, which imposes lower displacements of the interface portions manipulated by the surgeon of the master device body to obtain the desired degree of opening/closing gripping/cutting force, with respect to known solutions;
- in particular, by virtue of the mode of geometric variations between the sensors, as described above, a better reading resolution is provided for the tracking system of the master device;
- meanwhile, the positive sensation of control for the surgeon is also improved, by virtue of the fact that the first closed configuration of the master device body actually corresponds to a closed configuration of the slave device, and that therefore by increasing the clamping force on the master device, determining the second variation of the geometric relationship between the sensors, an increase in the clamping /cutting force of the slave device is obtained, intuitively;
- a closed master device corresponds to a closed slave device, while an open master device corresponds to an open slave device, and the modulation of the closing force occurs with the master device closed, intuitively for an operator;
- the shape of the master control device can be designed to maximize the familiarity of the surgeon or microsurgeon, and can be substantially a reproduction of traditional surgical or microsurgical instruments (i.e., to operate directly without robotic mediation).

. It is understood that the combinations of features of the appended claims form an integral part of the present description.

### LIST OF REFERENCE SIGNS

- 11: First operating portion of the master device
- 12: Second operating portion of the master device
- 13: Free end
- 14: Guide or constraint
- 15: Elastic device
- 16: Rotational constraint
- 17: Contact point
- 18: Elastic device
- 19: Curved or folded section
- 21: First manipulation portion of the master device
- 22: Second manipulation portion of the master device
- 23: Data cable
- 31: First tracking sensor
- 32: Second tracking sensor
- 33: Detector of the detector strip
- 34: Guide or track
- 35: Abutment and dragging surface
- 36: Rotating member
- 100: Robotic system for medical or surgical teleoperation
- 101: System for simulating medical or surgical teleoperation
- 104: System control unit
- 110: Master control device of the system for teleoperation, or master device
- 111: Support for the operating console of the master device
- 112: Master device joint
- 113: Wearable element of the master device
- 120: Slave assembly of the teleoperation system
- 127: Camera or microscope
- 128: Display
- 130: Slave robotic manipulator
- 140: Master console
- 142: Tracking field emitter
- 144: Master workspace
- 150: User, or operator, or surgeon
- 170: Slave surgical instrument
- 171: Slave surgical instrument tip or jaw
- 172: Slave surgical instrument tip or jaw
- 173: Slave surgical instrument rotational joint
- 174: Slave workspace
- 175: Operating portion of the tip or jaw
- 177: Control point
- 178: Body to be clamped
- MFO: Master global reference system
- SFO: Slave global reference system
- GR: Degree of freedom of opening/closing of the slave surgical instrument
- X1: First direction
- X2: Second direction
- X3: Third direction
- P1: End-of-stroke
- P2: End-of-stroke
- L: Longitudinal direction of the master device
- R, R: Rotation axis of the rotating member
- R2: Radius of the rotating member
- B: Orientation of the first closed configuration
- C: Orientation of the second closed configuration
- Y2: Distance
- Y: Angular stroke
- Z: Angular stroke
- F: Clamping/cutting force

## Claims

1. A robotic system (100) for medical or surgical teleoperation and/or simulation of a teleoperation, comprising:
- at least one slave surgical instrument (170) with at least one degree of freedom of opening/closing (GR),
- at least one master control device (110) for controlling at least the degree of freedom of opening/closing (GR) of the slave surgical instrument (170), said master control device comprising a body having a first operating portion (11) and second operating portion (12) which are mutually movable under the action imparted by the hand of a surgeon to take at least one open configuration, adapted to control at least one enslaved open configuration of the slave surgical instrument, and at least one closed configuration, adapted to control at least one enslaved closed configuration of the slave surgical instrument
said master control device further comprising a first manipulation portion (21) and a second manipulation portion (22) for the action imparted by the surgeon's hand; in which the first manipulation portion is in operating connection with the first operating portion of the body of the master device, and the second manipulation portion is in operating connection with the second operating portion of the master device body, in which the first manipulation portion and the second manipulation portion are mutually movable along a single third direction (X3) of relative approach/distancing;
wherein an elastic device is intended to influence the master control device (110) in an open configuration; and wherein the master device (110) further comprises the elastic device between the first manipulation portion (21) and the second manipulation portion (22) of the body of the master device (110), configured to influence the first manipulation portion (21) and the second manipulation portion (22) in mutual distancing;
said master control device (110) comprising a first tracking sensor (31) in operating connection with the first operating portion (11) and a second tracking sensor (32) in operating connection with the second operating portion (12);
and wherein, the system (100) further comprising at least one control unit (104), said at least one control unit (104) is configured to detect a pose and/or a relative movement of the tracking sensors (31,32), said at least one control unit (104) is configured to command to said at least one slave surgical instrument (170) an opening/closing movement and a modulation of the clamping/cutting force (F) based on the detected relative movement of the tracking sensors (31,32),
wherein an actuation of a control movement imposed by a user on the master control device (110) according to a single closing/opening movement trajectory (X3) results in:
- a first relative movement between the two sensors (31, 32) of the master control device (110) according to a first relative movement direction (X1), which is detected by the control unit, based on which the control unit commands the opening/closing movement of the slave surgical instrument (170) and a second relative movement between the same two sensors (31, 32) of the master control device (110) according to a second relative movement direction (X2), which is detected by the control unit, based on which the control unit exclusively commands to the slave surgical instrument (170) said clamping/cutting force modulation of the slave surgical instrument (170);
and wherein the control unit (104) is configured to:
- detect said first relative movement between the first tracking sensor (31) and the second tracking sensor (32) of the master control device (110) along said first relative movement direction (X1), wherein said first relative movement direction (X1) is related to a first geometric relationship between the first tracking sensor and the second tracking sensor,
- based on the first relative movement detected, move said slave surgical instrument (170) to open/close;
- detect said second relative movement between the first tracking sensor (31) and the second tracking sensor (32) of the master control device (110) along said second relative movement direction (X2), wherein the second relative movement direction (X2) is different from the first relative movement direction (X1), wherein the second relative movement direction (X2) is related to a second geometric relationship,
- based on the second relative movement detected, modulate the clamping/cutting force (F) of the slave surgical instrument (170);
and wherein the second variation of the geometric relationship between the first tracking sensor (31) and the second tracking sensor (32) is determined by the elastic deformation of the body of the master control device (110).

2. System (100) according to claim 1, wherein the control unit exclusively commands to the slave surgical instrument (170) said clamping/cutting force modulation of the slave surgical instrument (170) avoiding commanding the opening/closing movements of the slave instrument (170).

3. System (100) according to claim 1 or 2, wherein
- the first relative movement between the two sensors (31, 32) according to said first relative movement direction (X1) is a relative distance variation between the two sensors (31, 32),
- the second relative movement between the same two sensors (31, 32) according to said second relative movement direction (X2) is an orientation variation between the two sensors (31, 32).

4. A system (100) according to claim 1 or 2, wherein
- the first relative movement between the two sensors (31, 32) according to said first relative movement direction (X1) is a first defined curved trajectory and/or circumferential arc,
- the second relative movement between the same two sensors (31, 32) according to said second relative movement direction (X2) is a second, different defined curved trajectory and/or circumferential arc.

5. System (100) according to claim 4, wherein said second, different defined curve trajectory of said second relative movement direction (X2) [LS1] has a concavity opposite to said first defined curve trajectory of said first relative movement direction (X1).

6. System (100) according to claim 1 or 2 or 3, wherein the control unit (104) is configured to:
- detect a distance variation between the first tracking sensor (31) and the second tracking sensor (32),
- based on the distance variation detected, move the slave surgical instrument (170) to open/close;
- detect a relative orientation variation between the first tracking sensor (31) and the second tracking sensor (32),
- based on the relative orientation variation, modulate the clamping/cutting force (F) of the slave surgical instrument (170).

7. System (100) according to one of the preceding claims, wherein during the modulation of the clamping/cutting force (F) and/or during the second relative movement between the two sensors (31, 32) according to said second relative movement direction (X2), the control unit (104) is configured to inhibit any translation induced to the slave surgical instrument (170) or of a control point (177) caused by any further relative movement between the two sensors (31, 32) according to said first relative movement direction (X1).

8. System (100) according to claim 7, wherein by exerting said control movement in closing/opening according to said single movement trajectory (X3) of said master control device (110), no translation movements of the slave surgical instrument (170) or of the control point (177) are mechanically transferred and/or the midpoint calculated with the distance or the relative angle between the sensors (31, 32) is mechanically kept unchanged and constant.

9. System (100) according to one of the preceding claims, wherein
- by exerting said control movement in closing according to said single movement trajectory (X3), upon reaching and exceeding a contact point between two distinct sensorized portions of the master device (110) comprising said first sensor (31) and said second sensor (32), respectively, any further relative movement according to said first relative movement direction (X1) is mechanically inhibited, while only the movement according to said second relative movement direction (X2) is mechanically allowed,
- by exerting the control movement in opening according to said single movement trajectory (X3) upon reaching and exceeding said contact point, the relative movement according to said second relative movement direction (X2) is mechanically inhibited, while only the movement according to said first relative movement direction (X1) is mechanically allowed.

10. System (100) according to one of the preceding claims, wherein
- by exerting the control movement in closing according to said single movement trajectory (X3), upon reaching and exceeding a contact point between two distinct sensorized portions of the master device (110) comprising said first sensor (31) and said second sensor (32), respectively, and during the movement along said second relative movement direction (X2), it is necessary to exert a closing force (F2) on the master control device (110) which is greater than the force (F1) required to move the two sensorized portions of the master device (110) along said first relative movement direction (X1).

11. System (100) according to one of the preceding claims, wherein said single movement trajectory (X3) is a curve and/or a circumferential arc or is a straight line.

12. System (100) according to one of the preceding claims, wherein:
- a first part of the control movement according to said single movement trajectory (X3) imposed by the user on the master control device (110) is associated with the actuation of said first relative movement direction (X1) and thus with the actuation of the closing/opening of said slave surgical instrument (170);
- a second part of the control movement according to said single movement trajectory (X3) imposed by the user on the master control device (110) is associated with the actuation of said second relative movement direction (X2) and thus with the actuation of the modulation of the clamping/cutting force (F) of the slave surgical instrument (170).

13. System (100) according to one of the preceding claims, wherein the variation of the geometric relationship between the first tracking sensor (31) and the second tracking sensor (32) is amplified with respect to a variation of a geometric relationship between manipulation interface portions (21, 22) of the master control device (110), adapted to receive the fingers of an operator (150).

14. System (100) according to one of the preceding claims, wherein an elastic element (18) is provided between each manipulation portion (21, 22) and the respective operating portion (11, 12) provided with the respective tracking sensor (31, 32).

15. System (100) according to one of the preceding claims, wherein the tracking sensors are fixed to the respective operating portion (11, 12).

16. System (100) according to one of the preceding claims, wherein the master device can consist of two parts connected in an elastically preloaded connection joint, each part in turn consists of two rigid sections, a proximal and a distal section, connected by an elastic means, wherein the proximal section include portions or surface areas where the fingers rest and the distal part includes portions where tracking sensors (31, 32) can be inserted.

17. A control method for a system for simulating a medical or surgical teleoperation comprising the steps of:
- providing at least one master control device (110) as defined by claim 1 for controlling at least one degree of freedom of opening/closing (GR) of a simulation of a slave surgical instrument;
- detecting a first variation of a geometric relationship between the first tracking sensor (31) and the second tracking sensor (32) of the master control device (110) along a first direction (X1), and based on the first detected variation of the geometric relationship, moving the slave surgical instrument for simulation to open/close;
- detecting a second variation of the geometric relationship between the first tracking sensor (31) and the second tracking sensor (32) of the master control device (110) along a second direction (X2), based on the second detected variation of the geometric relationship, modulating a clamping/cutting force (F) of the slave surgical instrument for simulation.

18. A computer program configured to perform the steps of the method according to claim 17, when executed on a system (100) according to claim 1.

## Patentansprüche

1. Robotisches System (100) zur medizinischen oder chirurgischen Teleoperation und/oder zur Simulation einer Teleoperation, umfassend:
- mindestens ein chirurgisches Sklaveninstrument (170) mit mindestens einem Freiheitsgrad zum Öffnen/Schließen (GR),
- mindestens ein Hauptsteuergerät (110) zur Steuerung mindestens des Freiheitsgrads des Öffnens/Schließens (GR) des chirurgischen Sklaveninstruments (170), wobei das Hauptsteuergerät einen Körper umfasst, der einen ersten Bedienabschnitt (11) und eine zweiten Bedienabschnitt (12) aufweist, die unter der Wirkung, die durch die Hand eines Chirurgen vermittelt wird, gegeneinander beweglich sind, um mindestens eine offene Konfiguration einzunehmen, die eingerichtet sind, mindestens eine unterworfene offene Konfiguration des Sklaveninstruments zu steuern, und mindestens eine geschlossene Konfiguration, die eingerichtet ist, mindestens eine unterworfene geschlossene Konfiguration des Sklaveninstruments zu steuern,
das Hauptsteuergerät ferner umfassend:
einen ersten Manipulationsabschnitt (21) und einen zweiten Manipulationsabschnitt (22) für die Wirkung, die durch die Hand des Chirurgen vermittelt wird; wobei der erste Manipulationsabschnitt in Bedienverbindung mit dem ersten Bedienabschnitt des Körpers des Hauptgeräts steht und der zweite Manipulationsabschnitt in Bedienverbindung mit dem zweiten Bedienabschnitt des Körpers des Hauptgeräts steht, wobei der erste Manipulationsabschnitt und der zweite Manipulationsabschnitt gegeneinander entlang einer einzelnen dritten Richtung (X3) des betreffenden Annäherns/Entfernens beweglich sind; wobei eine elastische Vorrichtung dazu bestimmt ist, das Hauptsteuergerät (110) in einer offenen Konfiguration zu beeinflussen; und wobei das Hauptgerät (110) ferner die elastische Vorrichtung zwischen dem ersten Manipulationsabschnitt (21) und dem zweiten Manipulationsabschnitt (22) des Körpers des Hauptgeräts (110) umfasst, die dazu konfiguriert ist, den ersten Manipulationsabschnitt (21) und den zweiten Manipulationsabschnitt (22) in gegenseitiger Entfernung zu beeinflussen;
wobei das Hauptsteuergerät (110) einen ersten Verfolgungssensor (31) umfasst, der in Bedienverbindung mit dem ersten Bedienabschnitt (11) steht und einen zweiten Verfolgungssensor (32) der in Bedienverbindung mit dem zweiten Bedienabschnitt (12) steht;
und wobei das System (100) ferner mindestens eine Steuereinheit (104) umfasst, wobei die mindestens eine Steuereinheit (104) dazu konfiguriert ist, eine Position und/oder eine Relativbewegung der Verfolgungssensoren (31, 32) zu erfassen, wobei die mindestens eine Steuereinheit (104) dazu konfiguriert ist, dem mindestens einen chirurgischen Sklaveninstrument (170) eine Öffnungs-/Schließbewegung und eine Modulation der Klemm-/Schneidkraft (F) basierend auf der erfassten Relativbewegung der Verfolgungssensoren (31, 32) zu befehlen,
wobei eine Betätigung einer Steuerbewegung, die durch einen Benutzer an dem Hauptsteuergerät (110) nach einer einzelnen Öffnungs-/Schließbewegungstrajektorie (X3) ausgeübt wird, folgendes bewirkt:
- eine erste Relativbewegung zwischen den zwei Sensoren (31, 32) des Hauptsteuergeräts (110) nach einer ersten Relativbewegungsrichtung (X1), die von der Steuereinheit erfasst wird, basierend darauf die Steuereinheit die Öffnungs-/Schließbewegung des chirurgischen Sklaveninstruments (170) befiehlt und
eine zweite Relativbewegung zwischen denselben zwei Sensoren (31, 32) des Hauptsteuergeräts (110) nach einer zweiten Relativbewegungsrichtung (X2), die von der Steuereinheit erfasst wird, basierend darauf die Steuereinheit dem chirurgischen Sklaveninstrument (170) die Klemm-/Schneidkraftmodulation des chirurgischen Sklaveninstruments (170) befiehlt;
und wobei die Steuereinheit (104) dazu konfiguriert ist, um:
- die erste Relativbewegung zwischen dem ersten Verfolgungssensor (31) und dem zweiten Verfolgungssensor (32) des Hauptsteuergeräts (110) entlang der ersten Relativbewegungsrichtung (X1) zu erfassen, wobei die erste Relativbewegungsrichtung (X1) mit einer ersten geometrischen Beziehung zwischen dem ersten Verfolgungssensor und dem zweiten Verfolgungssensor in Zusammenhang steht,
- basierend auf der erfassten ersten Relativbewegung das chirurgische Sklaveninstrument (170) zu bewegen, um zu öffnen/schließen;
- die zweite Relativbewegung zwischen dem ersten Verfolgungssensor (31) und dem zweiten Verfolgungssensor (32) des Hauptsteuergeräts (110) entlang der zweiten Relativbewegungsrichtung (X2) zu erfassen, wobei die zweite Relativbewegungsrichtung (X2) von der ersten Relativbewegungsrichtung (X1) verschieden ist, wobei die zweite Relativbewegungsrichtung (X2) mit einer zweiten geometrischen Beziehung in Zusammenhang steht,
- basierend auf der erfassten zweiten Relativbewegung die Klemm-/Schneidkraft (F) des chirurgischen Sklaveninstruments (170) zu modulieren;
und wobei die zweite Änderung der geometrischen Beziehung zwischen dem ersten Verfolgungssensor (31) und dem zweiten Verfolgungssensor (32) durch die elastische Verformung des Körpers des Hauptsteuergeräts (110) bestimmt wird.

2. System (100) nach Anspruch 1, wobei die Steuereinheit ausschließlich dem chirurgischen Sklaveninstrument (170) die Klemm-/Schneidkraftmodulation des chirurgischen Sklaveninstruments (170) befiehlt, indem sie verhindert, die Öffnungs-/Schließbewegungen des Sklaveninstruments (170) zu befehlen.

3. System (100) nach Anspruch 1 oder 2, wobei
- die erste Relativbewegung zwischen den zwei Sensoren (31, 32) nach der ersten Relativbewegungsrichtung (X1) eine relative Abstandsänderung zwischen den zwei Sensoren (31, 32) ist,
- die zweite Relativbewegung zwischen denselben zwei Sensoren (31, 32) nach der zweiten Relativbewegungsrichtung (X2) eine Orientierungsänderung zwischen den zwei Sensoren (31, 32) ist.

4. System (100) nach Anspruch 1 oder 2, wobei
- die erste Relativbewegung zwischen den zwei Sensoren (31, 32) nach der ersten Relativbewegungsrichtung (X1) eine erste definierte gekrümmte Trajektorie und/oder ein Kreisbogen ist,
- die zweite Relativbewegung zwischen denselben zwei Sensoren (31, 32) nach der zweiten Relativbewegungsrichtung (X2) eine zweite, verschiedene definierte gekrümmte Trajektorie und/oder ein Kreisbogen ist.

5. System (100) nach Anspruch 4, wobei die zweite, verschiedene definierte Kurventrajektorie der zweiten Relativbewegungsrichtung (X2) [LS1] eine Konkavität aufweist, die der ersten definierten Kurventrajektorie der ersten Relativbewegungsrichtung (X1) entgegengesetzt ist.

6. System (100) nach Anspruch 1 oder 2 oder 3, wobei die Steuereinheit (104) dazu konfiguriert ist, um:
- eine Abstandsänderung zwischen dem ersten Verfolgungssensor (31) und dem zweiten Verfolgungssensor (32) zu erfassen,
- basierend auf der erfassten Abstandsänderung das chirurgische Sklaveninstrument (170) zu bewegen, um zu Öffnen/Schließen,
- eine relative Orientierungsänderung zwischen dem ersten Verfolgungssensor (31) und dem zweiten Verfolgungssensor (32) zu erfassen,
- basierend auf der relativen Orientierungsänderung die Klemm-/Schneidkraft (F) des chirurgischen Sklaveninstruments (170) zu modulieren.

7. System (100) nach einem der vorhergehenden Ansprüche, wobei während der Modulation der Klemm-/Schneidkraft (F) und/oder während der zweiten Relativbewegung zwischen den zwei Sensoren (31, 32) nach der zweiten Relativbewegungsrichtung (X2), die Steuereinheit (104) dazu konfiguriert ist, jede Verschiebung, die auf das chirurgische Sklaveninstrument verursacht wird oder eines Steuerpunkt (177), der durch eine weitere Relativbewegung zwischen den zwei Sensoren (31, 32) nach der ersten Relativbewegungsrichtung (X1) verursacht wird zu vermeiden.

8. System (100) nach Anspruch 7, wobei durch Ausübung der Steuerbewegung in Schließung/Öffnung nach der einzigen Bewegungstrajektorie (X3) des Hauptsteuergeräts (110) keine Verschiebungsbewegungen des chirurgischen Sklaveninstruments (170) oder des Steuerpunkts (177) mechanisch übertragen werden und/oder der Mittelpunkt, der mit dem Abstand oder dem relativen Winkel zwischen den Sensoren (31, 32) berechnet wird, mechanisch unverändert und konstant gehalten wird.

9. System (100) nach einem der vorhergehenden Ansprüche, wobei
- durch Ausübung der Steuerbewegung in Schließung nach der einzigen Bewegungstrajektorie (X3), bei Erreichen und Überschreiten eines Kontaktpunkts zwischen zwei verschiedenen sensorisierten Abschnitten des Hauptgeräts (110) umfassend den ersten Sensor (31) bzw. den zweiten Sensor (32), jede weitere Relativbewegung nach der ersten Relativbewegungsrichtung (X1) mechanisch vermeidet wird, während nur die Bewegung nach der zweiten Relativbewegungsrichtung (X2) mechanisch zugelassen wird,
- durch Ausübung der Steuerbewegung in Öffnung nach der einzigen Bewegungstrajektorie (X3) bei Erreichen und Überschreiten des Kontaktpunkts, die Relativbewegung nach der zweiten Relativbewegungsrichtung (X2) mechanisch vermeidet wird, während nur die Bewegung nach der ersten Relativbewegungsrichtung (X1) mechanisch zugelassen wird.

10. System (100) nach einem der vorhergehenden Ansprüche, wobei
- durch Ausübung der Steuerbewegung in Schließung nach der einzigen Bewegungstrajektorie (X3), bei Erreichen und Überschreiten eines Kontaktpunkts zwischen zwei verschiedenen sensorisierten Abschnitten des Hauptgeräts (110), umfassend den ersten Sensor (31) und den zweiten Sensor (32), und während der Bewegung entlang der zweiten Relativbewegungsrichtung (X2), eine Schließkraft (F2) auf das Hauptsteuergerät (110) ausgeübt werden muss, die größer als die Kraft (F1) ist, die erforderlich ist, um die zwei sensorisierten Abschnitte des Hauptgeräts (110) entlang der ersten Relativbewegungsrichtung (X1) zu bewegen.

11. System (100) nach einem der vorhergehenden Ansprüche, wobei die einzige Bewegungstrajektorie (X3) eine Kurve und/oder ein Kreisbogen oder eine Gerade ist.

12. System (100) nach einem der vorhergehenden Ansprüche, wobei:
- ein erster Teil der Steuerbewegung nach der einzigen Bewegungstrajektorie (X3), die durch den Benutzer auf das Hauptsteuergerät (110) ausgeübt wird, der Aktivierung der ersten Relativbewegungsrichtung (X1) und somit der Aktivierung der Öffnung/Schließung des chirurgischen Sklaveninstruments (170) zugeordnet ist,
- ein zweiter Teil der Steuerbewegung nach der einzigen Bewegungstrajektorie (X3), die durch den Benutzer auf das Hauptsteuergerät (110) ausgeübt wird, der Aktivierung der zweiten Relativbewegungsrichtung (X2) und somit der Aktivierung der Modulation der Klemm-/Schneidkraft (F) des chirurgischen Sklaveninstruments (170) zugeordnet ist.

13. System (100) nach einem der vorhergehenden Ansprüche, wobei die Änderung der geometrischen Beziehung zwischen dem ersten Verfolgungssensor (31) und dem zweiten Verfolgungssensor (32) in Bezug auf einer Änderung einer geometrischen Beziehung zwischen Manipulationsschnittstellenabschnitten (21, 22) des Hauptsteuergeräts (110), die eingerichtet sind, die Finger eines Bedieners (150) aufzunehmen, verstärkt wird.

14. System (100) nach einem der vorhergehenden Ansprüche, wobei ein elastisches Element (18) zwischen jedem Manipulationsabschnitt (21, 22) und dem jeweiligen Bedienabschnitt (11, 12), der mit dem jeweiligen Verfolgungssensor (31, 32) versehen ist, vorgesehen ist.

15. System (100) nach einem der vorhergehenden Ansprüche, wobei die Verfolgungssensoren an den jeweiligen Bedienabschnitt (11, 12) befestigt sind.

16. System (100) nach einem der vorhergehenden Ansprüche, wobei das Hauptgerät aus zwei Teilen bestehen kann, die durch ein elastisch vorgespanntes Verbindungsgelenk verbunden sind, wobei jeder Teil wiederum aus zwei starren Abschnitten besteht, einem proximalen und einem distalen Abschnitt, die durch ein elastisches Mittel verbunden sind, wobei der proximale Abschnitt Abschnitte oder Flächenbereiche enthält, auf denen die Finger aufliegen, und der distale Teil Abschnitte enthält, in die Verfolgungssensoren (31, 32) eingesetzt werden können.

17. Steuerverfahren für ein System zur Simulation einer medizinischen oder chirurgischen Teleoperation, umfassend die folgenden Schritte:
- Bereitstellen mindestens eines Hauptsteuergerätes (110) nach Anspruch 1 zur Steuerung von mindestens einem Freiheitsgrad der Öffnung/Schließung (GR) einer Simulation eines chirurgischen Sklaveninstruments;
- Erfassen einer ersten Änderung einer geometrischen Beziehung zwischen dem ersten Verfolgungssensor (31) und dem zweiten Verfolgungssensor (32) des Hauptsteuergeräts (110) entlang einer ersten Richtung (X1), und basierend auf der ersten erfassten Änderung der geometrischen Beziehung, Bewegen des chirurgischen Sklaveninstruments für die Simulation zum Öffnen/Schließen,
- Erfassen einer zweiten Änderung der geometrischen Beziehung zwischen dem ersten Verfolgungssensor (31) und dem zweiten Verfolgungssensor (32) des Hauptsteuergeräts (110) entlang einer zweiten Richtung (X2), basierend auf der zweiten erfassten Änderung der geometrischen Beziehung, Modulieren einer Klemm-/Schneidkraft (F) des chirurgischen Sklaveninstruments für die Simulation.

18. Computerprogramm, das dazu konfiguriert ist, die Schritte des Verfahrens nach Anspruch 17 auszuführen, wenn es auf einem System (100) nach Anspruch 1 ausgeführt wird.

## Revendications

1. Un système robotique (100) pour téléopération médicale ou chirurgicale et/ou simulation d'une téléopération, comprenant :
au moins un instrument chirurgical esclave (170) avec au moins un degré de liberté d'ouverture/fermeture (GR),
au moins un dispositif maître de commande (110) pour commander au moins le degré de liberté d'ouverture/fermeture (GR) de l'instrument chirurgical esclave (170), ledit dispositif maître de commande comprenant un corps ayant une première partie de commande (11) et une seconde partie de commande (12) mutuellement mobiles sous l'action exercée par la main d'un chirurgien pour adopter au moins une configuration ouverte, adaptée pour commander au moins une configuration ouverte esclave de l'instrument chirurgical esclave, et au moins une configuration fermée, adaptée pour commander au moins une configuration fermée esclave de l'instrument chirurgical esclave
ledit dispositif maître de commande comprenant en outre
une première portion de manipulation (21) et une seconde portion de manipulation (22) pour l'action exercée par la main du chirurgien ; dans lequel la première portion de manipulation est en liaison de fonctionnement avec la première partie de commande du corps du dispositif maître, et la seconde portion de manipulation est en liaison de fonctionnement avec la seconde partie de commande du corps du dispositif maître, dans lequel la première portion de manipulation et la seconde portion de manipulation sont mutuellement mobiles le long d'une unique troisième direction (X3) d'un rapprochement/éloignement relatif ;
dans lequel un dispositif élastique est destiné à influencer le dispositif maître de commande (110) dans une configuration ouverte ; et dans lequel ledit dispositif maître (110) comprend le dispositif élastique entre la première portion de manipulation (21) et la seconde portion de manipulation (22) du corps du dispositif maître (110), configuré pour influencer la première portion de manipulation (21) et la seconde portion de manipulation (22) dans un éloignement mutuel ;
ledit dispositif maître de commande (110) comprenant un premier capteur de suivi (31) en liaison de fonctionnement avec la première partie de commande (11) et un second capteur de suivi (32) en liaison de fonctionnement avec la seconde partie de commande (12);
et dans lequel le système (100) comprend en outre au moins une unité de commande (104), ladite au moins une unité de commande (104) étant configurée pour détecter une pose et/ou un mouvement relatif des capteurs de suivi (31,32), ladite au moins une unité de commande (104) étant configurée pour commander audit au moins un instrument chirurgical esclave (170) un mouvement d'ouverture/fermeture et une modulation de la force de serrage/coupe (F) en fonction du mouvement relatif détecté des capteurs de suivi (31,32),
dans lequel une action d'un mouvement de commande imposé par un utilisateur sur le dispositif maître de commande (110) selon la trajectoire de mouvement de fermeture/ouverture (X3) entraîne :
un premier mouvement relatif entre les deux capteurs (31, 32) du dispositif maître de commande (110) selon une première direction de mouvement relatif (Xl), qui est détecté par l'unité de commande,
- sur la base de quoi l'unité de commande commande le mouvement d'ouverture/fermeture de l'instrument chirurgical esclave (170) et
un second mouvement relatif entre les mêmes deux capteurs (31, 32) du dispositif maître de commande (110) selon une seconde direction de mouvement relatif (X2), qui est détecté par l'unité de commande, sur la base de quoi l'unité de commande commande exclusivement à l'instrument chirurgical esclave (170) ladite modulation de la force de serrage/coupe de l'instrument chirurgical esclave (170) ;
et dans lequel l'unité de commande (104) est configurée pour :
- détecter ledit premier mouvement relatif entre le premier capteur de suivi (31) et le second capteur de suivi (32) du dispositif maître de commande (110) le long dudit premier direction de mouvement relatif (Xl), dans lequel ladite première direction de mouvement relatif (Xl) est liée à une première relation géométrique entre le premier capteur de suivi et le second capteur de suivi,
- sur la base du premier mouvement relatif détecté, déplacer ledit instrument chirurgical esclave (170) pour ouvrir/fermer ;
- détecter ledit second mouvement relatif entre le premier capteur de suivi (31) et le second capteur de suivi (32) du dispositif maître de commande (110) le long dudit second direction de mouvement relatif (X2), dans lequel la seconde direction de mouvement relatif (X2) est différente de la première direction de mouvement relatif (Xl), dans lequel la seconde direction de mouvement relatif (X2) est liée à une seconde relation géométrique,
- sur la base du second mouvement relatif détecté, moduler la force de serrage/coupe (F) de l'instrument chirurgical esclave (170) ;
et dans lequel la seconde variation de la relation géométrique entre le premier capteur de suivi (31) et le second capteur de suivi (32) est déterminée par la déformation élastique du corps du dispositif maître de commande (110).

2. Système (100) selon la revendication 1, dans lequel l'unité de commande commande exclusivement à l'instrument chirurgical esclave (170) ladite modulation de la force de serrage/coupe de l'instrument chirurgical esclave (170) en évitant de commander les mouvements d'ouverture/fermeture de l'instrument esclave (170).

3. Système (100) selon la revendication 1 ou 2, dans lequel
le premier mouvement relatif entre les deux capteurs (31, 32) selon ladite première direction de mouvement relatif (Xl) est une variation de distance relative entre les deux capteurs (31, 32),
le second mouvement relatif entre les mêmes deux capteurs (31, 32) selon ladite seconde direction de mouvement relatif (X2) est une variation d'orientation entre les deux capteurs (31, 32).

4. Système (100) selon la revendication 1 ou 2, dans lequel
le premier mouvement relatif entre les deux capteurs (31, 32) selon ladite première direction de mouvement relatif (Xl) est une première trajectoire définie courbe et/ou un arc circonférentiel,
le second mouvement relatif entre les mêmes deux capteurs (31, 32) selon ladite seconde direction de mouvement relatif (X2) est une seconde trajectoire définie courbe différente et/ou un arc circonférentiel.

5. Système (100) selon la revendication 4, dans lequel ladite seconde trajectoire courbe définie différente de ladite seconde direction de mouvement relatif (X2) [LS1] a une concavité opposée à ladite première trajectoire courbe définie de ladite première direction de mouvement relatif (Xl).

6. Système (100) selon la revendication 1 ou 2 ou 3, dans lequel l'unité de commande (104) est configurée pour :
- détecter une variation de distance entre le premier capteur de suivi (31) et le second capteur de suivi (32),
- sur la base de la variation de distance détectée, déplacer l'instrument chirurgical esclave (170) pour ouvrir/fermer ;
- détecter une variation d'orientation relative entre le premier capteur de suivi (31) et le second capteur de suivi (32),
- sur la base de la variation d'orientation relative, moduler la force de serrage/coupe (F) de l'instrument chirurgical esclave (170).

7. Système (100) selon l'une quelconque des revendications précédentes, dans lequel pendant la modulation de la force de serrage/coupe (F) et/ou pendant le second mouvement relatif entre les deux capteurs (31, 32) selon ladite seconde direction de mouvement relatif (X2), l'unité de commande (104) est configurée pour inhiber toute translation induite à l'instrument chirurgical esclave (170) ou à un point de commande (177) causée par tout autre mouvement relatif entre les deux capteurs (31, 32) selon ladite première direction de mouvement relatif (X1).

8. Système (100) selon la revendication 7, dans lequel, en exerçant ledit mouvement de commande en fermeture/ouverture selon ladite trajectoire de mouvement unique (X3) dudit dispositif maître de commande (110), aucun mouvement de translation de l'instrument chirurgical esclave (170) ou du point de commande (177) n'est mécaniquement transféré et/ou le point médian calculé avec la distance ou l'angle relatif entre les capteurs (31, 32) est mécaniquement maintenu inchangé et constant.

9. Système (100) selon l'une quelconque des revendications précédentes, dans lequel
en exerçant ledit mouvement de commande en fermeture selon ladite trajectoire de mouvement unique (X3), lors de l'atteinte et du dépassement d'un point de contact entre deux portions distinctes instrumentées du dispositif maître (110) comprenant respectivement ledit premier capteur (31) et ledit second capteur (32), tout mouvement relatif supplémentaire selon ladite première direction de mouvement relatif (X1) est mécaniquement inhibé, tandis que seul le mouvement selon ladite seconde direction de mouvement relatif (X2) est mécaniquement autorisé,
en exerçant le mouvement de commande en ouverture selon ladite trajectoire de mouvement unique (X3), lors de l'atteinte et du dépassement dudit point de contact, le mouvement relatif selon ladite seconde direction de mouvement relatif (X2) est mécaniquement inhibé, tandis que seul le mouvement selon ladite première direction de mouvement relatif (X1) est mécaniquement autorisé.

10. Système (100) selon l'une quelconque des revendications précédentes, dans lequel
en exerçant le mouvement de commande en fermeture selon ladite trajectoire de mouvement unique (X3), lors de l'atteinte et du dépassement d'un point de contact entre deux portions distinctes instrumentées du dispositif maître (110) comprenant respectivement ledit premier capteur (31) et ledit second capteur (32), et pendant le mouvement le long de ladite seconde direction de mouvement relatif (X2), il est nécessaire d'exercer une force de fermeture (F2) sur le dispositif maître de commande (110) qui est supérieure à la force (F1) requise pour déplacer les deux portions instrumentées du dispositif maître (110) le long de ladite première direction de mouvement relatif (X1).

11. Système (100) selon l'une quelconque des revendications précédentes, dans lequel ladite trajectoire de mouvement unique (X3) est une courbe et/ou un arc circonférentiel ou est une ligne droite.

12. Système (100) selon l'une quelconque des revendications précédentes, dans lequel :
une première partie du mouvement de commande selon ladite trajectoire de mouvement unique (X3) imposée par l'utilisateur sur le dispositif maître de commande (110) est associée à l'activation de ladite première direction de mouvement relatif (X1) et ainsi à l'activation de l'ouverture/fermeture dudit instrument chirurgical esclave (170) ;
une seconde partie du mouvement de commande selon ladite trajectoire de mouvement unique (X3) imposée par l'utilisateur sur le dispositif maître de commande (110) est associée à l'activation de ladite seconde direction de mouvement relatif (X2) et ainsi à l'activation de la modulation de la force de serrage/coupe (F) de l'instrument chirurgical esclave (170).

13. Système (100) selon l'une quelconque des revendications précédentes, dans lequel la variation de la relation géométrique entre le premier capteur de suivi (31) et le second capteur de suivi (32) est amplifiée par rapport à une variation d'une relation géométrique entre les portions d'interface de manipulation (21, 22) du dispositif maître de commande (110), adaptées à recevoir les doigts d'un opérateur (150).

14. Système (100) selon l'une quelconque des revendications précédentes, dans lequel un élément élastique (18) est prévu entre chaque portion de manipulation (21, 22) et la portion de commande respective (11, 12) pourvue du capteur de suivi respectif (31, 32).

15. Système (100) selon l'une quelconque des revendications précédentes, dans lequel les capteurs de suivi sont fixés à la portion de commande respective (11, 12).

16. Système (100) selon l'une quelconque des revendications précédentes, dans lequel le dispositif maître peut être constitué de deux parties reliées par une articulation à connexion préchargée élastiquement, chaque partie étant à son tour constituée de deux sections rigides, une section proximale et une section distale, reliées par un moyen élastique, la section proximale comprenant des portions ou des zones de surface sur lesquelles reposent les doigts et la partie distale comprenant des portions où peuvent être insérés les capteurs de suivi (31, 32).

17. Procédé de commande pour un système de simulation d'une téléopération médicale ou chirurgicale comprenant les étapes de :
fournir au moins un dispositif maître de commande (110) tel que défini dans la revendication 1 pour commander au moins un degré de liberté d'ouverture/fermeture (GR) d'une simulation d'un instrument chirurgical esclave ;
détecter une première variation d'une relation géométrique entre le premier capteur de suivi (31) et le second capteur de suivi (32) du dispositif maître de commande (110) le long d'une première direction (X1), et sur la base de la première variation détectée de la relation géométrique, déplacer l'instrument chirurgical esclave pour simulation pour ouvrir/fermer ;
détecter une seconde variation de la relation géométrique entre le premier capteur de suivi (31) et le second capteur de suivi (32) du dispositif maître de commande (110) le long d'une seconde direction (X2), sur la base de la seconde variation détectée de la relation géométrique, moduler une force de serrage/coupe (F) de l'instrument chirurgical esclave pour simulation.

18. Programme informatique configuré pour exécuter les étapes du procédé selon la revendication 17, lorsqu'il est exécuté sur un système (100) selon la revendication 1.
